(19)

Europäisches
Patentamt

European
Patent Office

Office européen
des brevets

(11)     **EP 1 959 880 B1**

(12)               **EUROPEAN PATENT SPECIFICATION**

(45) Date of publication and mention
     of the grant of the patent:
     **26.09.2012  Bulletin 2012/39**

(51) Int Cl.:
     **A61F 5/03** *(2006.01)*        **A61F 13/00** *(2006.01)*

(21) Application number: **06818149.4**

(22) Date of filing: **05.12.2006**

(86) International application number:
     **PCT/DK2006/000690**

(87) International publication number:
     **WO 2007/065435 (14.06.2007 Gazette 2007/24)**

(54) **GARMENT  FOR  ABDOMINAL  COMPRESSION**

HÜLLE  FÜR  BAUCHKOMPRESSION

VETEMENT  POUR  COMPRESSION ABDOMINALE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **06.12.2005  DK 200501731
            16.12.2005  DK 200501782
            13.02.2006  DK 200600207
            05.04.2006  US 789148 P
            24.08.2006  DK 200601098
            29.11.2006  DK 200601572**

(43) Date of publication of application:
     **27.08.2008  Bulletin 2008/35**

(73) Proprietor: **TYTEX A/S
     7430 Ikast (DK)**

(72) Inventors:
• **SOERENSEN, Balslev, Bettina
  DK-8600 Silkeborg (DK)**
• **JENSEN, Hedevang, Svend, Erik
  DK-7430 Ikast (DK)**
• **BIRK, Jonna
  DK-7400 Herning (DK)**
• **SKOV, Verstergaard, Charlotte
  DK-8653 Them (DK)**
• **ÖZDEMIR, Cengiz
  DK-7430 Ikast (DK)**

(74) Representative: **Hoffmann Dragsted A/S
     Rådhuspladsen 16
     1550 Copenhagen V (DK)**

(56) References cited:
     **US-A1- 2004 127 881     US-A1- 2005 215 964
     US-B1- 6 194 629**

EP 1 959 880 B1

## Description

### Field of the Invention

The present invention is related to the field of hernia care and prevention and, more particularly, to a post-operative garment with particular elastic properties for the prevention of hernias, as defined in claim 1.

### Background

Hernia is an abnormal protrusion of an organ or parts thereof through the wall of a body cavity. A special type of hernia is an incisional (ventral) hernia that may appear when persons have undergone surgery, where the incision goes through various tissues, and the tissue is thus weakened by the incision. The incision can be any cut or hole made in the skin possibly extending through the fascia. It includes midline, transverse, oblique or other incisions made during laparotomy, key-holes made during laparoscopy and trocar sites. The incidence of incisional hernia varies from a few percent to more than 30% depending on the reasons for surgery (diagnosis), operation procedures used and general wound healing ability of the patient. Incisional hernia can appear a relatively long time after the surgery, i.e., months or even years. The recurrence rate of hernia can be very high.

To protect the hernia incision after surgery, compression garments can be worn. These are particularly important to reduce the risk of wound healing complications relating to temporary increases in intraabdominal pressure. Intraabdominal pressure (IAP) is defined as the pressure concealed within the abdominal cavity and varies with respiration. Sudden large increases in IAP occurs i.e. when the user sneezes, coughs, moves, vomits or defecates as well as during heavy lifting and exercise. During such a peak in IAP the wound is subjected to a significant shear force, tending to pull the wound edges apart which may result in increased scar formation, wound rupture (dehiscence), later incisional hernia formation or other wound healing complications.

While compression garments can help protect the wound during healing, patients often find them uncomfortable. As a result, patients often do not comply with post-operative instructions, choosing instead to remove the garment and thereby increasing the risk of healing complications.

An example of a fitting garment for preventing spread of operative wound is known from WO0024349 which discloses a garment having support bodies made of a non-slip material sandwiching the operative wound and having non-stretchable panels fixed to the support bodies on their outer surface. The free ends of the panels are joined to each other over the wound so as to draw the support bodies towards the wound thereby preventing spread of the operative wound.

FR2039950 discloses a bandage made of elastic material which has a layer of non-stretchable material fixed at the abdominal region. The bandage further comprises three reinforcements adapted for attaching two straps supporting the groin area.

US-A-2004-127881 discloses a garment of a material having a Linear elastic section and a progressive elastic section

A need still exists for an effective hernia prevention and treatment device that provides the necessary compression while, at the same time, providing comfort and adjustability so that wearer compliance with post-operative instructions calling for the consistent wearing of such a device is increased.

### Summary

The present invention is directed to a compression garment providing protection to a wound area or as a hernia prevention garment, which at the same time is comfortable to wear so as to increase compliance.

An object of the invention is to provide a garment having a supportive section of a material with a controlled stretch-profile for covering the wound or hernia area and where the garment has another section of an elastic material for providing the necessary compression and comfort for the patient. The supportive section of a material with a controlled stretch-profile provides a plate effect around the wound, so as to counteract further stretch in the material covering the wound.

A further object of the invention is to provide different garments having a supportive section and another section of an elastic material. Examples of the different garments are an abdominal binder or tube, an undershirt, underpants and a bodysuit.

The invention also provides a hernia prevention regimen using a garment with the two different materials sections.

**Detailed Disclosure**

A major aspect of the invention relates to a compression garment comprising two elastic sections, a linear elastic section being made of a material with linear elastic properties, and a progressive elastic section being made of a material with progressive elastic properties wherein the progressive elastic section below 20% extension has linear elastic material properties.

The boundary for the transition in the materials properties of the progressive elastic section may preferably be anywhere between 15 % and 25 % elongation, e.g. 16%, 17%, 18%, 19%, 20%, 21%, 22%, 23% or 24%. It may even be as low as 10% or as high as 30%. Another wording for this boundary is lower strain limit.

The present invention may be worn by anyone recovering from hernia repair, trying to prevent hernia formation or other certain surgeries. Certain patient groups have a higher than average risk of wound rupture or hernia formation and will especially benefit from using the present invention. Such high-risk groups include the obese, diabetics, smokers, patients with chronic obstructive lung disease (COLD) and patients with abnormal collagen I/III ratio (e.g. patient suffering from abdominal aorta aneurysm). However, all patient groups will benefit from the comfort and support offered by the invention which reduces post-operative pain during movement and provides the patient with an increased sense of security, i.e. allowing an earlier mobilization of the patient. Fast mobilization of patients is the most important factor in avoiding post-operative complications and reducing lengths of hospital stay.

In the long-term perspective, the risk of developing incisional hernia or recurrence of a repaired hernia will be reduced, when using the garment of the invention.

Not only will the product according to the invention reduce the wound healing complications caused by sudden increases in IAP (Intra Abdominal Pressure), but a constant pressure is also applied to the wound area reducing the formation of edema, seroma and hematoma as known, e.g., from compression stockings.

An important part of the present invention relates to a supportive section of the compression garment with a controlled stretchability. If elastic fabric is placed on the wound there is a risk that the fabric will tend to tear the wound/ bandage and stress the wound when the patient moves or is moved or handled by the caregiver. In contrast, when a supportive fabric with a controlled stretchability protects the wound, there is virtually no transverse stress.

The invention is based on the realization that a non-stretchable section of material is excellent in preventing wound rupture or hernia recurrence, but would cause complications due to abdominal compartment syndrome and would also be very uncomfortable to wear. An elastic or stretchable section of material would however not be able to prevent wound rupture or hernia recurrence but would provide the comfort and compression needed as well as allowing the necessary movement of the abdominal region. Therefore, a combination of a supportive section having a controlled stretchability with an elastic section would provide the necessary protection of the wound area while at the same time provide the necessary comfort for the user.

Another advantage is that a garment of a given size with such a supportive section provides a higher compression than a similar all-elastic garment of the same size. Thus, the supportive section provides a firm compression, which resembles a plate effect. This allows for a more elastic textile, and thus one that is more comfortable, and/or a smaller elongation compared to an all-elastic garment.

In an embodiment the supportive section (the progressive elastic section) reaches a maximum level of stretch where further stretch is impossible. Such a supportive section may be referred to as a substantially non-stretchable part or section.

In one embodiment of the invention the compression garment is a garment where the progressive elastic section is made of a material with non-linear elastic properties which has an increasing MoE within an extension of 20% - 40% and which material is non-stretchable with an extension above 40% with a brittle rupture at increased force and where the force required for rupture is higher than 0.5N/mm.

The upper boundary for the transition between non-linear elastic material properties to non-stretchable material may be anywhere between 30% and 50%, e.g. 35%, 36%, 37%, 38%, 39%, 40%, 41%, 42%, 43%, 44% or 45%.

Another embodiment of the compression garment is a garment where the progressive elastic section is made of a material with different linear elastic properties depending on the extension which material within an extension above 20% has linear elastic material properties with an MoE at least three times higher than the MoE of the material within an extension below 20%.

The MoE of the progressive elastic section above 20% elongation may be a factor of 2 to 10 greater than the MoE of the section below 20% elongation, e.g. a factor 3, 4, 5, 6, 7, 8 or 9.

The linear elastic section provides the elasticity needed to obtain compression, and at the same time making the garment able to be donned.

The progressive elastic section provides the support and protection of the wound area or the area likely to develop a hernia rupture. This is due to the fact that the progressive elastic section has a controlled extension profile meaning that the material is able to extend relatively easy up to 20 % extension after which the resistance to extension is more pronounced. Eventually further extension may be impossible. The consequence of this extension profile is that a patient wearing a compression garment according to the invention would be able to breath and would not experience discomfort due to a totally inelastic garment section. However, when a sudden large increase in IAP occurs, the garment is able to control the movement of the abdominal wall in the weak area surrounding the wound or hernia, thereby preventing wound rupture and a recurrence of hernia. Example 5 illustrates the difference in strain in the progressive elastic (supportive) and the elastic section.

By a progressive elastic section that covers an incision is essentially meant a homogeneous, supportive section of the garment. Prior art garments might have non-stretchable parts in the shape of zippers, seams, plates etc., but these are not suitable for the present invention which is directed to a supportive section with a controlled stretchability, preferably textile, that covers a wound arising from an incision. Such a part cannot comprise a zipper, for example, as that structure would conflict with the healing process and the sensitive skin around the wound.

The combination of the two elastic sections provides a unique garment, which at the same time is comfortable to wear and provides the necessary protection for the weak area of the abdominal wall.

In one embodiment the progressive elastic section of the material is made of a layered material where the layers have different material properties, a first layer having linear elastic material properties and another layer being slightly oversized compared to the first layer. This layered structure makes it possible to carefully control the extension profile of the material as the degree of over-sizing determines at which extension point the influence of the material properties of the oversized layer begins. This corresponds to the transition at the lower strain limit.

It is preferred that the first layer of the layered material corresponds to the material of the linear elastic section of the garment. This makes it possible to conceal the supportive progressive elastic section in the garment hence making the garment look like a regular non-supportive garment.

By selecting the oversized layer to be made of a virtually non-stretchable fabric or a fabric with a much higher MoE, e.g. three times the material of the first layer it is possible to control the stretchability of the layered structure.

The oversized layer has to be attached at both sides to the circular tube or binder of the compression garment. This ensures that expansion of the tube or binder in the radial direction does not cause noticeable expansion of the part of the garment intended to protect the wound. If the oversized layer were attached in only one side, the garment would act as if the supportive section was not present. The oversized layer may be attached by detachment components.

If the oversized layer is made of a virtually non-stretchable fabric it is preferred that the material has holes so it is possible to slightly deform the material. This makes the transition between the influence from the first and second layer of the garment less pronounced and hence the garment will be more comfortable to wear.

Preferably the oversized layer has holes corresponding to 25 -50 % of the area of the oversized layer, more preferred 30%, 35%, 40% or 45%.

A suitable yarn for making such a non-stretchable material is polyamide filament yarn.

The linear elastic material in the linear elastic section of garment has an MoE of 0.2-1.0 N/mm where the MoE is defined as illustrated in Example 1. An MoE of this order enables the material to be stretched easily while at the same time allowing the garment to provide the desired degree of compression to the patient.

The linear elastic section may have zones with different linear elastic properties thereby providing a three-dimensional effect and assuring an anatomic fit.

The extension in the linear elastic section should be linear at least up to 100% extension. This makes the garment able to easily stretch to at least twice the circumference, which makes it much easier to be donned.

To provide the necessary compression, it is preferred that the compression is between 1 mmHg and 40 mmHg. To be sure to avoid occurrence of abdominal compartment syndrome when the garment is used at a compression garment in the abdominal area, it is preferred that the compression is below 25 mmHg. To prevent hernia or wound rupture it is preferred that the compression is between 5 mmHg and 15 mmHg or even more preferred between 7 mmHg and 13 mmHg. Examples 1, 3 and 6 show calculations of compression in garments according to the invention. Example 6 provides the calculations showing the maximum compression to be below 25 mmHg even when a wrong size is used. When the right size is used the compression is between 5 and 13 mmHg at a strain of 10-30 % in the supportive section and a strain of 20-100% in the elastic section.

All types of garments according to the invention comprise a circular knit tube.

In one embodiment of the invention, the compression garment is in the shape of an undershirt. The undershirt preferably has comfort fabric in the upper part of the undershirt, as donning would be very hard if the entire garment were made from the compression providing material. To make donning easier, the garment comprises sections with easily stretchable fabric. The undershirt has a lower part providing compression and an upper part of easily stretchable fabric. The whole undershirt is preferably knitted in one process and there is preferably a seamless transition from the compression lower part to the easily stretchable upper part. With this design the undershirt can be donned more easily, especially by stepping into the garment and pulling it upwardly from beneath. Example 3 illustrates the compression as a result of wearing an undershirt according to this aspect of the invention. In another embodiment of the invention the garment is in the shape of an abdominal binder. In a preferred embodiment, the binder is higher in the front than it is in the back. Examples 3 and 6 illustrate the elastic properties of the abdominal binder.

In one embodiment the abdominal binder comprises a closure mechanism, which could be any means of closure well known in the art including zippers, straps, VELCRO, hooks and loops, buttons, magnets as well as hooks and eyes. Disposable closing mechanisms such as e.g. adhesive tapes can also be used.

In the early stages after surgery, the wound needs to be examined and there is a risk of bleeding and infection. Thus, wearing a compression garment during this time requires that the wound be easily accessible. However, the movements required to remove conventional clothing will increase the risk that wound healing is affected and increases the risk of wound complications and rupture. Thus, in a preferred embodiment of the invention, the supportive section, or parts thereof, is removable. In a related embodiment, the supportive section, or parts thereof, is replaceable. This is desirable, for example, if the part should become contaminated e.g. by blood.

When the supportive section, or parts thereof, is replaceable, the tube portion of the compression garment may be used for a longer period of time and is both more economical, easier for the caregiver and more comfortable to the patient, as lifting or rolling the patient is avoided. It is preferred that the supportive section is attached to the compression garment by any means of closure mechanisms like the ones used in connection with the abdominal binder. A preferred example of such closures, or fixation components, is a hook and eye combination.

The supportive section (which may be the front section) may further comprise elastic straps at the upper and lower corners. These straps make the detachment and attachment of the section to the tube part easier, as they can be used as "handles".

Another way of making detachment and attachment easier is to provide the abdominal binder with finger holes.

The garment may also be made in the shape of underpants or boxer briefs. The underpants or briefs may have comfort fabric in the lower part of the pants and the transition between the compression fabric and the comfort fabric may be seamless as it is described with the undershirt. The underpants may be worn alone or in combination with an undershirt or an abdominal binder, depending on the position and length of the incision.

Optimal hernia prevention may require wearing the undershirt and the underpants together.

In another embodiment the underpants may extend upwards towards the lower chest area. In a related embodiment, the underpants have an opening in the crotch region, allowing the user to go to the toilet without removing the garment in the incision area.

In another embodiment of the invention the compression garment is in the form of an abdominal tube, which may be entirely seamless. The tube is like the binder except that the initial knitted seamless tube has not been cut and provided with the hook and loop closure. Otherwise the tube may be provided with the same supportive section as the binder. The tube may further be provided with easily extendible elastic trims with a silicon pattern on the side facing the skin of the user.

Examples 3 and 6 illustrate calculations of the compression in the abdominal tube.

A high level of comfort is obtained with the compression garment of the invention by a substantially seamless design. That is, the garment, or at least the tubular part thereof, is knitted in one-piece. When worn while lying down, it is uncomfortable to rest on seams.

A bodysuit design is also an option, and so are high underpants extending to the lower chest region.

It is preferred that the compression garment underpants have a similar seamless transition from compression fabric to easily stretchable fabric, e.g. between the body encircling portion to the legs of the underpants and the crotch area, in order to avoid excess pressure to these areas.

The present invention is appropriate for use in connection with a broad range of incision types. One kind of incision is a Caesarian section. Here, a suitable garment is in the form of pants with a transversely oriented, supportive section in one or the other side of the front of the pants. For patients with a midline Caesarean, the pants will have a longitudinally oriented, supportive section in the middle of the front.

Removal of the appendix can be made with an oblique incision. Here, an appropriate garment is in the form of an undershirt having a supportive section on the right hand side of the front covering the incision. The section may have an oblique orientation.

Many procedures that commonly are or were performed in open surgery can today be performed as laparoscopic surgery, which is a method by which surgery is done by making small incisions on the abdominal wall and inserting the instruments through specially designed ports. Laparoscopic surgery is commonly used for removal of the gall bladder, removal of the appendix, tubal ligation (sterilisation) and hernia repair (incisional and other). Here, a desired garment is in the form of an undershirt, possibly in combination with a pair of pants, where one or more square supportive sections cover the incisions. Such a garment can also be used after diagnostic laparoscopy.

In the typical use, especially early after surgery, the incision site is first covered with a wound dressing. This wound dressing will absorb exudates from the wound, and might even promote healing through suitable additives.

Another example is patients operated on because of abdominal aortic aneurysm (AAA). This type of surgery often involves a long midline incision and patients have a high risk of incisional hernia formation. Here, a compression garment is either an abdominal binder or a combination of underpants and undershirt, where a supportive section covers the whole incision.

The supportive section may have a 'soft' inner surface. In one preferred embodiment, the section is formed from a piece of so-called "spacer fabric", which is a three layer fabric construction with a knitted inner side, a knitted outer side and pile yarns such as monofilament yarns forming pillars between the two sides. With this construction, the spacer fabric acts as a pressure distributing material and ensures an overall higher average compression compared to a garment made from compression fabric only.

In one embodiment of the present invention where the garment is a tube-like garment, the supportive section is built into the garment in the form of a front panel of such tube. In other embodiments of the invention, where the garment is an undershirt or underpants, the section is attached, i.e. sewn in, for example, on the inside of the front of the undershirt or underpants. This makes the supportive section non-visible, and makes the garment look essentially like normally underwear.

An important aspect in hernia prevention is that a garment for hernia prevention is to be used as soon as possible after the operation. This sets specific requirements relating to e.g. handling by health care personnel, monitoring of the compression level etc. The Abdominal binders according to the present invention can be worn essentially directly after surgery. Consequently, in one embodiment of the invention, the prevention regimen for hernia starts with a compression garment shaped as an abdominal binder.

In one embodiment the supportive section does not have hook and loops closure but is permanently attached to the tube portion, i.e. by sewing. Another embodiment is a tube-like garment with only one row of hooks and loops along one side of the supportive section. This enables opening and access to the wound, but not replacement of the supportive section separately.

The abdominal tube provides for support and compression, it is very simple and inexpensive to produce, has a compelling streamlined look but still features the protective supportive section.

The supportive section is preferably made of breathable material allowing the wound to heal. At the same time, the material is also preferably water permeable and/or vapor permeable and/or non-absorptive to wound liquids. The supportive section may further have transparent or semi-transparent properties for easy monitoring of the wound. In one embodiment this is achieved by using a mesh inner- or outer surface on the supportive section. This makes the garment useful as a medical bandage.

In order for the supportive section to cover the incision and effectively reduce shear forces on the incision/wound, it is preferable that the supportive section extends more than 1cm beyond the wound region such as more than 2cm wide, that is more than 3cm, or 4cm, or 5cm or 6cm or 7cm or 8cm, or 9cm, or 10cm, or even wider than 11 cm. This means that the width of the supportive section is at least 6 cm, more preferably at least 10 cm, maybe at least 15 cm or even at least 20 cm. Preferably the width is less than 40 cm and between 15 and 25 cm.

The supportive section may have any shape but is preferably of a shape similar to the wound region. In this way maximum comfort as well as proper protection of the wound is obtained. Whatever the size of the supportive section, it can be readily interchanged with a supportive section of any other size to provide a wide range of adjustability while retaining the same compression garment.

In a preferred embodiment the garment comprises a mechanism for measuring the elongation of the garment, preferably the radial elongation. As the elongation is substantially proportional to the compression obtained, elongation provides an effective monitoring tool for a nurse or the patient, as it is possible to read the compression of the garment. It is important to avoid a too high compression and to assure adequate compression. As the circumference around the abdomen can increase over a relatively short period of time due to, e.g., edema formation after surgery, this can result in an increase in compression. Providing the possibility to monitor the elongation, it is also possible to consider options as regards the increased compression, and one can, for example, choose to change the garment to a garment of a larger size and/or insert a larger non-stretchable part and thus reduce the compression accordingly. This is also an advantage in relation to patient weight gain/loss, or abdominal distension where a change in size of the garment may be appropriate.

Alternatively, if the garment is adjustable in size the monitoring of the elongation can be used for proper adjustment. In a particularly preferred embodiment the mechanism for measuring the elongation of the garment is an easily visible portion, i.e. a colored thread, knitted directly in the tubular item forming a line of a predetermined length, in a transverse direction relative to the tube. The formed line can, for example, be 10 cm long, when the garment lies flat on a plane surface. For example, an elongation to 14 cm (an increase of 40%) may indicate a level of compression recommended by the patient's doctor. A nurse or the patient himself may be provided with a sort of measuring device, such as a simple ruler, indicating the acceptable interval of compression when compared to the colored line in the garment, when the patient is wearing the garment.

When the garment is in the shape of a tube or a binder it is preferred that the garment has an easily extendible elastic trim. In a related embodiment the upper and/or lower part of the circular knit tube further includes silicon in a pattern so as to enhance friction between the garment and the skin under the elastic trim.

The invention can used in a method of preventing hernia or wound rupture for patients with an incision in the abdominal region with a compression garment comprising a circular knit tube with supportive sections (progressive elastic sections) and elastic sections forming a tube wall. The method includes:

(a) applying a compression garment as an abdominal binder after surgery, such that the supportive section is placed over the incision; and

(b) maintaining compression over the incision for up to 1 year through compression garments such as abdominal binders, underpants and/or undershirts such that the supportive section is placed where the incision was made.

The method of preventing hernia may also be known as a hernia prevention regimen.

In connection with a hernia prevention regimen it is of particular interest that the supportive section of the binder or tube is entirely detachable and thus it is possible to change only the part that covers the wound or incision. This part is most likely to become soiled by wound exudate so it may be necessary to change it more often than the rest of the binder or tube.

It may also be preferred to exchange one size of supportive section with another larger or smaller part thereby changing the compression in the garment. The changing of the supportive section may be done without removing the binder or tube, which is an advantage if the patient is temporarily immobilized.

The garment according to the invention is useful for prevention of hernia. Not only is it important to have external compression of the abdomen after abdominal surgery to reduce the incidence of incisional hernia, but in order that the preventive effect is obtained, the patient needs to wear the compression garment 24 hours a day for several months or even years. Interruptions during shower and change of garment are obviously needed. If user compliance is reduced, so is the preventive effect of the garment. To achieve high compliance the garment needs to be comfortable and to have a look that invites the patient to use it without looking like a patient. The preferred seamless knit tube is one aspect in providing comfort.

When the patient is mobilized, further needs arise. First of all, the patient is typically more mobile, for which reason the garment(s) for hernia prevention is preferably designed to allow the user to move around freely, including going to the toilet while the garment(s) stays in place. Discretion, comfort and ease of use are of particular importance. Thus, one embodiment relates to a regimen using a compression garment shaped as an undershirt.

**Brief Description of the Drawings**

Figure 1 illustrates a hernia prevention garment in the form of an abdominal binder having an elastic section and a supportive section in accordance with the present invention.

Figure 2A shows the elastic portion of the hernia prevention garment of figure 1.

Figure 2B shows supportive section of the hernia prevention garment of figure 1.

Figure 3A shows an abdominal binder and the location and orientation of six fabric samples subjected to tensile testing to demonstrate the elastic properties of the corresponding hernia prevention garment in accordance with the present invention.

Figure 3B is an enlarged view of one of the fabric samples of figure 3A.

Figure 4 shows average force-elongation curves from tensile testing of fabric samples 1 and 6 of figure 3A.

Figure 5 shows average force-elongation curves from tensile testing of fabric samples 1&6, 2&5, and 3&4 of figure 3A.

Figures 6A and 6B illustrate front and back view, respectively, of a hernia prevention garment in the form of a compression undershirt in accordance with the present invention.

Figures 7A and 7B illustrate front and back view, respectively, of a hernia prevention garment in the form of compression boxer briefs for men in accordance with the present invention.

Figure 8A illustrates an embodiment of an abdominal binder in accordance with the present invention, shown in the open position.

Figure 8B illustrates a front view of the abdominal binder of figure 8A in a closed position.

Figure 8C illustrates a rear view of the closed abdominal binder of figure 8B.

Figure 9A illustrates yet another embodiment of the abdominal binder which has a more pronounced figure-shape such that the back part of the binder is significantly lower than the front part, shown in the open position.

Figure 9B illustrates a front view of the abdominal binder of figure 9A in a closed position.

Figure 9C illustrates a rear view of the closed abdominal binder of figure 9B.

Figures 10A and 10B illustrate inside and outside view, respectively, of the cuts and stitches which constitutes the finger holes for the abdominal binder.

Figure 11 illustrates the principle of tensile testing.

Figure 12 illustrates the relationship between the major and minor axes in the ellipsoid waist as a function of the waist size.

Figure 13 illustrates the obtained force/strain curves for the elastic fabric, Corsinel.

Figure 14 illustrates the linear regression used on the measured force/strain relationship shown in figure 13.

Figure 15 illustrates the difference between the elastic section and the supportive section as illustrated by stress-strain curves.

Figure 16 illustrates the obtained force/strain curves for a layered materials fabric of Corsinel and non-stretchable material

Figure 17 illustrates the best polynomial fit used on the measured force/strain relationship shown in figure 15.

Figure 18 illustrates the calculations for the layered material, the elastic material and the tube.

Figure 19 illustrates the strain in the layered material as a function of the overall strain.

Figure 20 illustrates the comparison between a tube made entirely of elastic material and a tube made from a combination of elastic and supportive, layered material.

**Detailed Description of the Preferred Embodiments.**

In describing a preferred embodiment of the invention illustrated in the drawings, specific terminology will be resorted to for the sake of clarity. However, the invention is not intended to be limited to the specific terms so selected, and it is to be understood that each specific term includes all technical equivalents which operate in a similar manner to accomplish a similar purpose.

For materials characteristics of the materials mentioned in the description of the figures, reference is made to example 2.

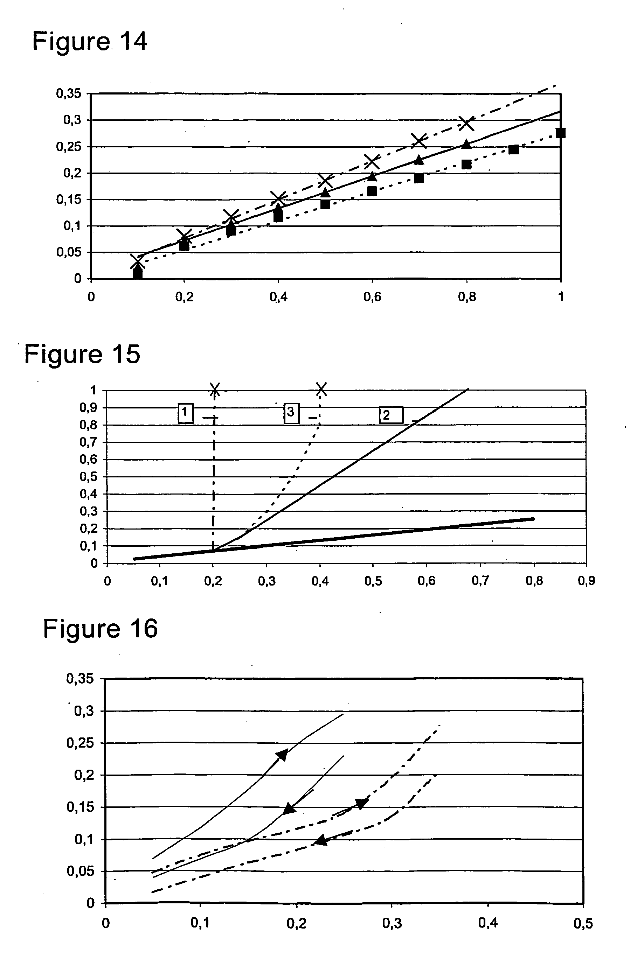

A material with progressive elastic properties is defined as a material having a non-constant and increasing MoE and where the MoE for higher extensions leaves the material section with a high resistance to further stretch or maybe even non-stretchable. For definitions of MoE used in this application, see example 1. This indicates that the material section would stretch relatively freely up to a certain level (the lower strain limit) after which further stretch will be virtually impossible. This is clear when compared to the extendibility of the elastic section, as described in the following, see figure 15 comparing the elastic and different embodiments of the supportive section represented by curves 1-3.

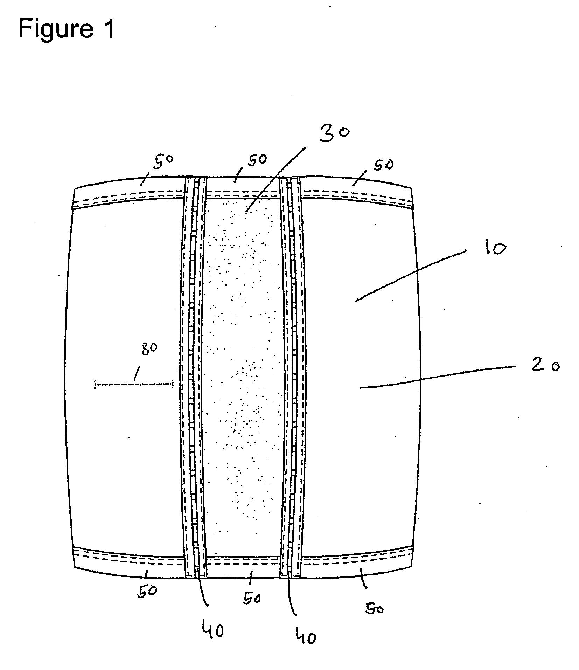

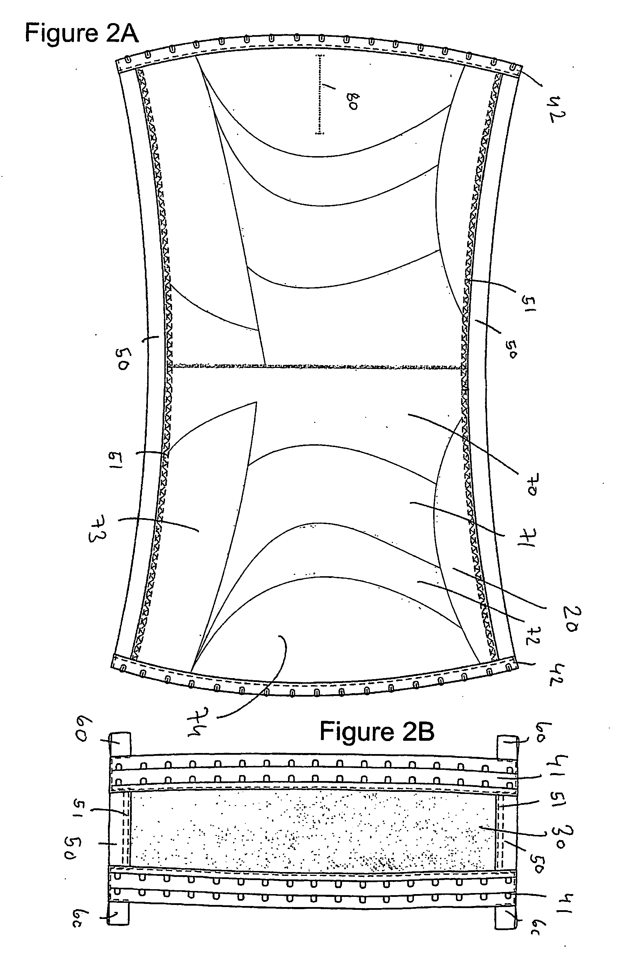

Figures 1, 2A and 2B show an abdominal binder 10 including a circular knit tube 20 made from elastic compression material C and a supportive section or front piece 30 made from spacer fabric material A. In figure 1, the front piece 30 is attached to the tube 20, while figures 2A and 2B illustrate the tube and front piece, respectively, as detached from one another.

The front piece 30 can be connected to the elastic tube 20 by means of front closures 40. In the shown embodiment two row polyamide/polypropylene bands with covered metal eyes 41 are sewn together with the spacer fabric front piece. Corresponding polyamide/polypropylene bands with covered metal hooks 42 are sewn into the edges 39 of the elastic tube 20. In a preferred embodiment, a vertical separation of 2.5 cm between adjacent hooks on the band is chosen which is larger than usually employed but ensures that the closure remains closed during use but still is relatively fast to close. Including a vertically extending reinforcement element within the edges 43 of the front piece 30 could provide greater spacing.

The horizontal separation of the metal eyes is about 2 cm, providing a total adjustment of about 4 cm and wider separation distances could also provide for greater adjustment.

Closure components 41 are attached to both sides of the front piece. This provides the opportunity to access the wound from the most convenient side and to change the front piece without having to change the whole binder and thereby having to move or roll the patient over.

The upper and lower edges of the tube 20 and front piece 30 are provided with 25 mm wide, easily extendible elastic trims 50 with silicon dots or pattern on the side facing the skin of the user. Such silicon dots provide a comfortable and non-occlusive means of keeping the garment in place during use. In the embodiment shown in figures 1, 2A and 2B, the elastic trims 50 are sewn 51 into the tube or front piece. Elastic straps 60 have been sewn into the front piece to provide "handles" as a means of keeping the textile in place when attaching and detaching the tube. The straps 60 stay on the inside of the garment and, when the front piece is attached to the tube, the straps 60 can be pulled on to stretch the tube.

To obtain a three-dimensional effect, assuring an anatomic fit, the tube is knitted with different zones 70-74 of varying stitch density. The back zone 70 of fabric variant C2 is the tighter structure, followed by transition zones 71, 72, towards the more elastic front zone 74 of fabric variant C1. Front zone 73 has an intermediate density providing support to the abdomen of the user.

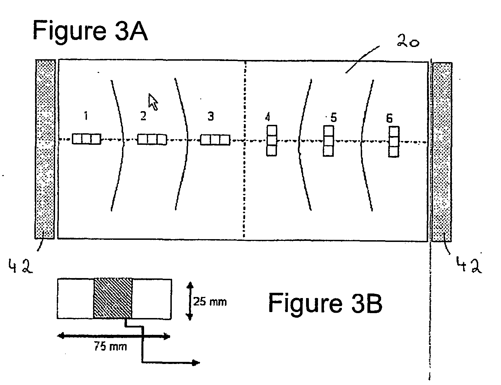

The material and the different zones are characterized by their elastic properties, which can be measured by tensile testing as is well known in the art. Fabric samples as located in different zones are illustrated in figure 3A, with an enlarged view of one of the samples being illustrated in figure 3B.

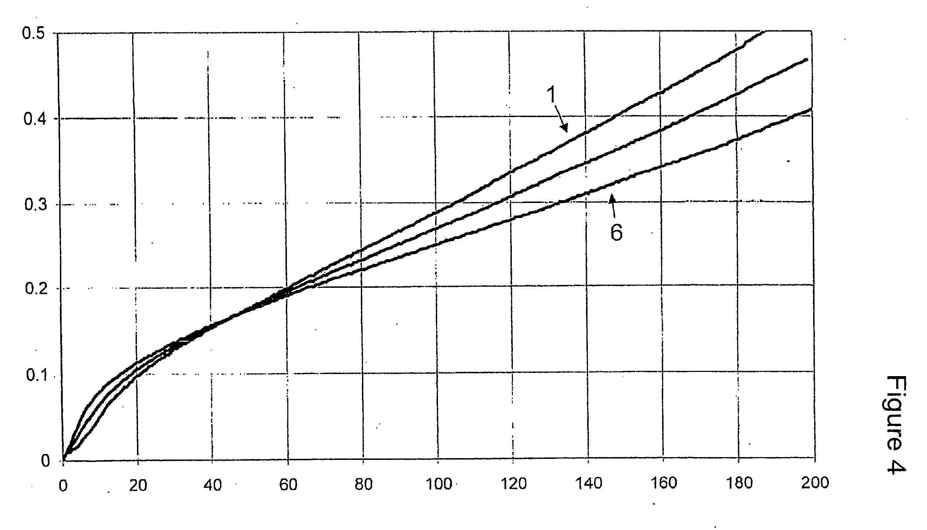

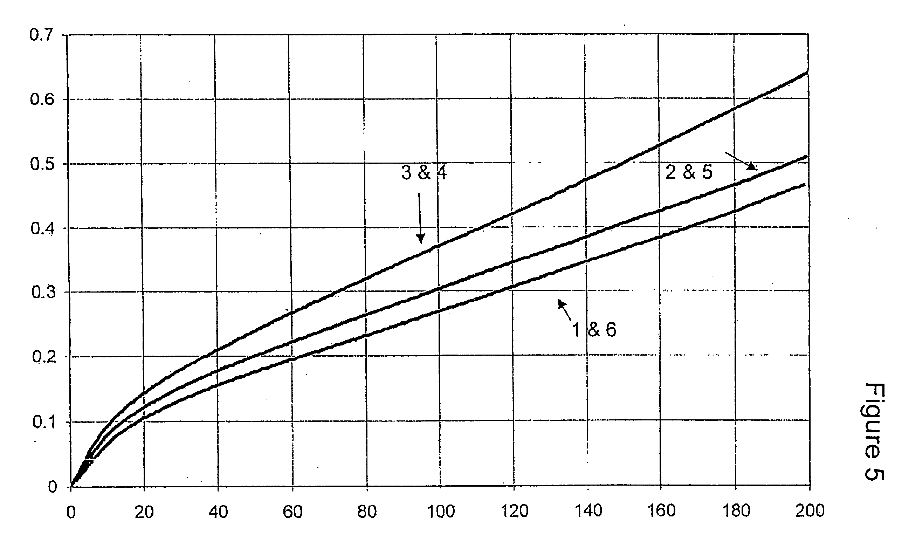

Finally, the binder is provided with a knitted-in coloured line 80 in a specific length, e.g., 10 cm. When the binder is in use, the elongation of the fabric can be read off directly from the elongation of the line. The measured elongation can then be combined with tensile test results as shown in figures 4 and 5 to estimate the compression provided by the binder.

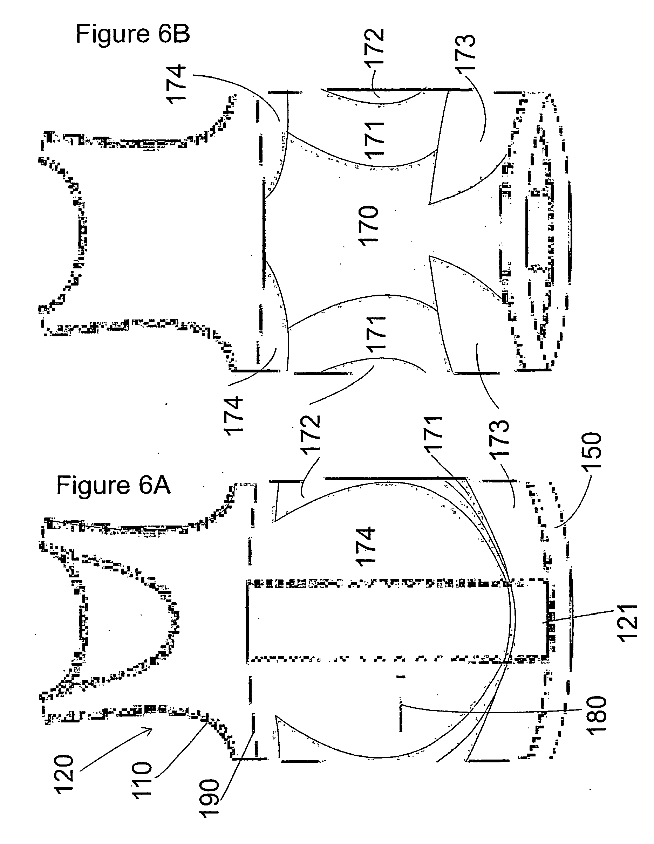

Figures 6A and 6B show front and back views, respectively, of a compression undershirt 100 designed to get as close as possible to the look of a normal undershirt. The shirt is circularly knit in one piece and includes an upper part 110 made of material D and a lower part 120 made of material C. On the front of the lower part 120 as shown in figure 6A, a fabric piece 121 of material B is sewn to the inside of the lower part 120. The fabric piece 121 is substantially non-stretchable and has a function similar to the substantially non-stretchable front piece 30 of the abdominal binder of figure 1, but remains thin and invisible from the outside.

The lower part of the undershirt is made from material C with different zones 170 - 174 of varying elasticity and density similar to zones 70 - 74 of the abdominal binder. Zone 170 is made from fabric variant C2, and zone 174 is made from fabric variant C1, with other zones 171, 172, 173 being intermediate as discussed in connection with zones 71, 72, 73 of the abdominal binder. The undershirt is provided at the lower portion of the lower part 120 with an elastic trim 150 with silicon dots or pattern on the side facing the skin of the user, and a knitted-in colored line 180 for measurement of the fabric elongation.

The undershirt includes a seamless transition 190 from the low density, essentially easily stretchable, comfort fabric D of the upper part 110 to the dense and highly elastic compression fabric C of the lower part 120. The comfort fabric of the upper part 110 allows the undershirt to be donned from beneath, like a women's one-piece bathing suit. An undershirt made only from highly elastic compression fabric cannot be donned in any reasonable way without an opening mechanism such as a zipper, which is highly uncomfortable for the user and very hard to zip due to the compression power of the fabric.

The combination of an easily stretchable upper part 110 and a compression lower part 120 allows the undershirt to be easily donned and provides a look very close to normal underwear.

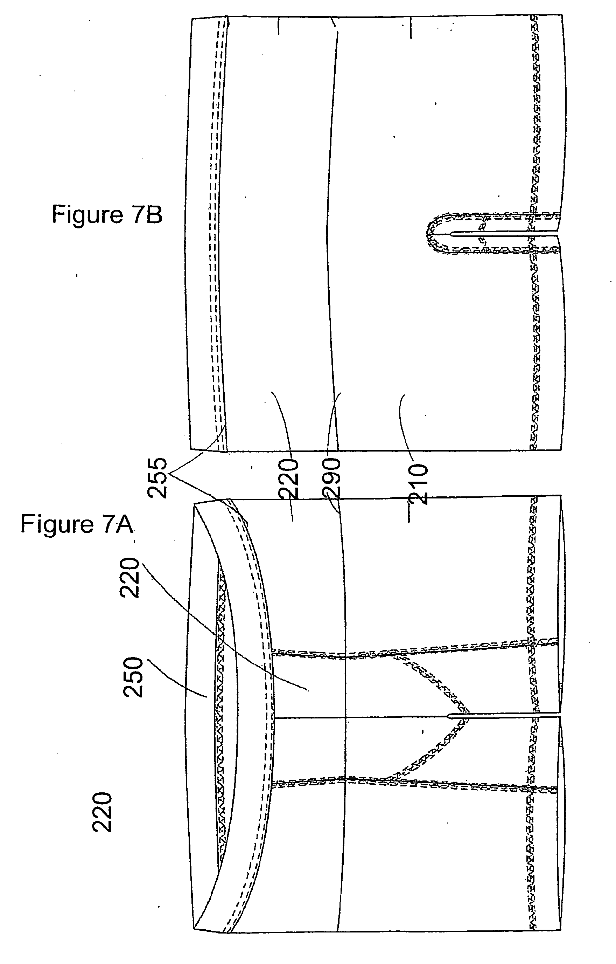

Figures 7A and 7B show front and back views, respectively, of a pair of male underpants 200 made as a pair of boxer briefs (with legs). The underpants 200 include an upper part 220 of compression fabric C and a lower part 210 of comfort fabric D with a seamless transition 290 between the two fabrics. The look is made as close as possible to normal underwear. The pants are provided with a fabric piece 221 of essentially non-stretchable material B sewn to the inside of the upper part 220. This non-stretchable fabric piece 221 serves the same purpose as the substantially non-stretchable fabric piece 121 of the undershirt 100.

The front waistline 255 of the pants is positioned lower than the back waistline 256 and even lower than in normal underpants. This ensures that the pants will be placed below the stomach of a user. It may be an advantage as the stomach of the user, for newly operated patients, may be distended or swollen. When the pants 200 are used together with the undershirt 100 of figure 6, the undershirt 100 covers the whole abdomen and provides compression even when the pants are removed during visits to the toilet.

The pants are provided at the top end thereof with an elastic trim 250 with silicon dots or pattern on the side facing the skin of the user.

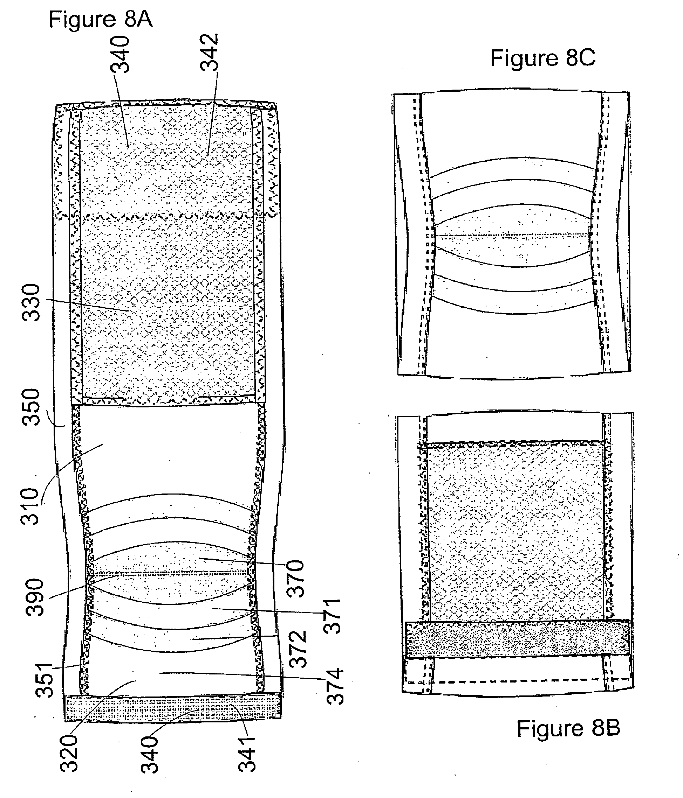

Figure 8A shows an abdominal binder 310 in the open position including a circular knit tube 320 made from elastic compression material C and a substantially non-stretchable front piece 330 made from spacer fabric material A. Figures 8B and 8C provide front and rear views, respectively, of the binder 310 in the closed position as when worn.

The front piece 330 can be connected to the elastic tube 320 by means of front closures 340. In the shown embodiment, a hook and loop system is used with a 38 mm wide hook material 341, such as male VELCRO, attached to the elastic compression material and a loop material 342, such as female VELCRO, attached to the supportive section or front piece 330.

The upper and lower edges of the tube 320 and front piece 330 are provided with 25 mm wide, easily extendible, elastic trims 350 with silicon dots or pattern on the side facing the skin of the user. Such silicon dots or pattern provide a comfortable and non-occlusive means of keeping the garment in place during use. In the embodiment shown in figures 8A, 8B and 8C, the elastic trims are sewn 351 into the tube and/or front piece.

To obtain a three-dimensional effect, assuring an anatomic fit, the tube is knitted with different zones 370, 371, 372, 374 of varying stitch density. The back zone 370 of fabric variant C2 is the tightest structure, followed by transition zones 371, 372 towards the more elastic front zone 374 of fabric variant C1.

The material and the different zones are characterized by their elastic properties, which can be measured by tensile testing as is well known in the art, see example 1.

Finally, the binder is provided with a knitted-in colored line 390 marking the centerline on the back of the binder. This feature is useful when the user is bed-ridden and health-care personnel apply the binder.

These figures discloses a binder allowing large adjustment in size, compression and fit by means of the hook and loop closure solution. There is no distinction between the upper and lower edges of the binder according to this embodiment, i.e., the binder can be worn with either edge "up", enabling the hook and loop closure to be placed on the left or right part of the abdomen as the user wishes.

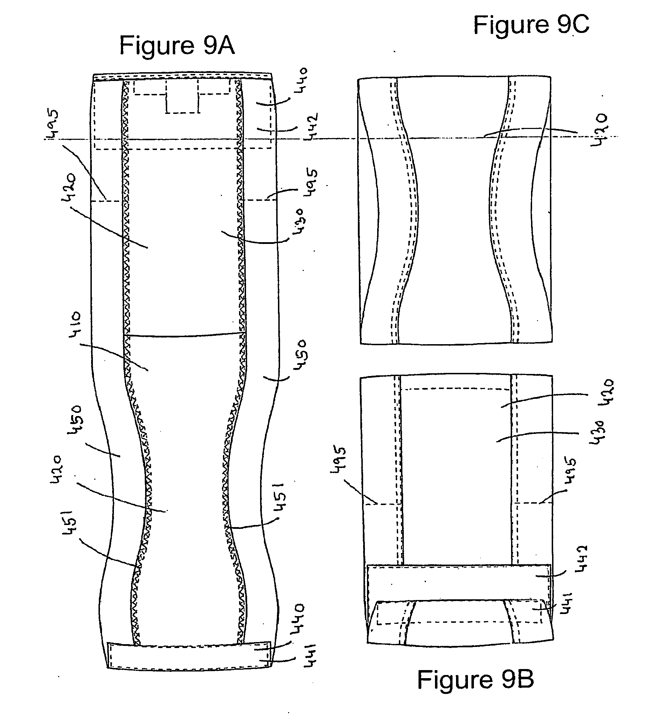

Figure 9A shows an abdominal binder 410 in the open position consisting of a circular knit tube 420 made from elastic compression material C (see example 2 for material characteristics). Figures 9B and 9C provide front and rear views, respectively, of the binder 410 in the closed position as when worn. On the front of the tube 430 a piece of material B is sewn to the inside of the tube. This piece of material B is substantially non-stretchable and has a function similar to the supportive section or front piece 330 of e.g. the abdominal binder of figure 8A, but remains thin and invisible from the outside.

The binder can be opened and closed, and the size adjusted by means of front closures 440. In the shown embodiment a hook and loop system is used with a hook material (male Velcro) 441 attached to one end of the elastic compression material and a loop material 442 attached to the other end.

The upper and lower edges of the tube 410 are provided with 35 mm wide easily extendible elastic trims 450 with a silicon pattern on the side facing the skin of the user. Such a silicon pattern provides a comfortable and non-occlusive means of keeping the garment in place during use. In the embodiment shown in figure 9A, the elastic trims 451 are sewn into the tube.

This embodiment is made using only one stitch density (fabric variant C1) of the tube material 420. However, a three-dimensional effect can easily be obtained using different zones of varying stitch densities as described e.g. in the reference of figure 8A.

The tube material is characterized by its elastic properties, which can be measured by tensile testing as well known in the art, see example 1.

The binder is provided with a knitted-in colored line 495 marking the centerline on the front of the binder. This feature is useful when applying the binder ensuring that the substantially non-stretchable protective fabric is placed correctly over the incision, wound or dressing.

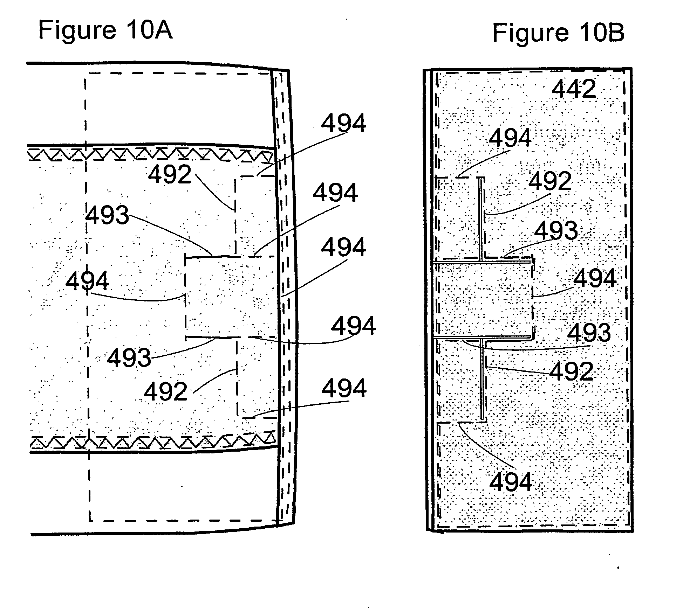

Finally, this binder is provided with finger holes made by cuts 492, 493 and stitches 494 in the loop material 442 as shown in figures 10A and B. These finger holes are useful for applying the binder. The holes made by cuts 492 will most likely be used by a user applying the binder him self, the holes made by cuts 493 will most likely be used by health care personal applying the binder on a patient. As indicated in figure 10A the cuts and stitches are nearly invisible from the outside. Figure 10B shows the cuts and stitches from the inside where the finger holes are visible.

This binder allows large adjustment in size, compression and fit by means of the hook and loop closure solution. There is no up and down of the binder of this embodiment, hence, the hook and loop closure can be placed on the left or right part of the abdomen as the user wishes.

Instead of a binder a tube may be provided. The tube may be entirely seamless and is similar to the binder except for the hook and loop closure of the binder, which is lacking in the tube. Like the different binders, the tube is also provided with the supportive section made of fabric B (see example 2 for materials characteristic) sewn to the inside of the front of a knitted tube made of elastic compression material C. The tube is furthermore provided with easily extendible elastic trims with a silicon pattern on the side facing the skin of the user.

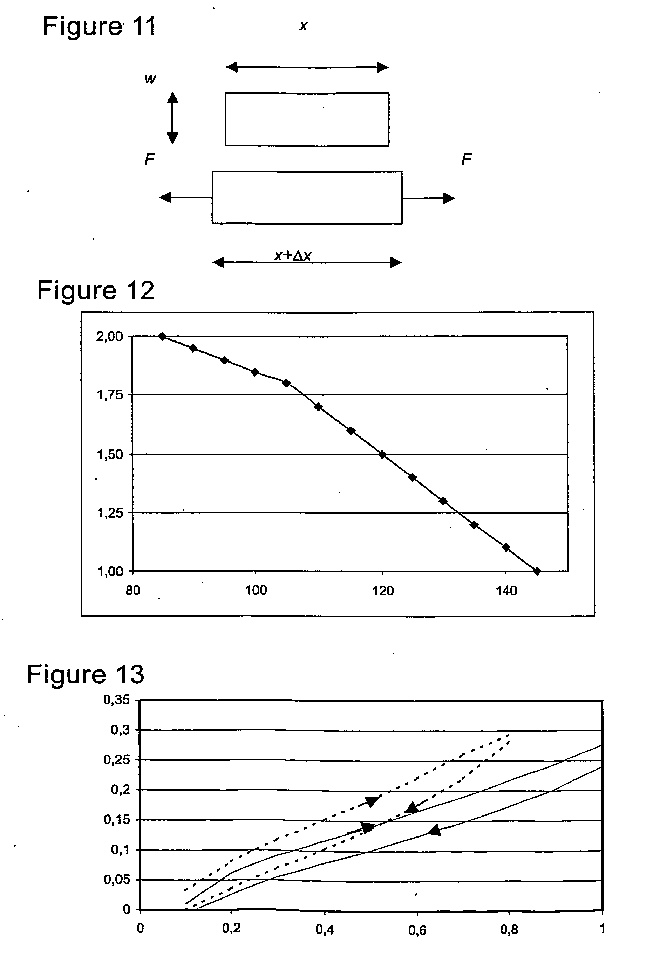

Figure 11 illustrates the principle of tensile testing used for the experiments on the tube. Tensile testing is performed by applying a given force (F) to the material and measuring the elongation (Δx) of the material. Prior to application of force (F) the dimensions of the sample is width (w) times length (x). When force (F) is applied the length is increased to x+ Δx.

Figure 12 shows the assumed relationship between waist size and ellipsoidal axes a and b. The Y-axis is the major ellipsoidal axis a in proportion to the minor ellipsoidal axis b - this is dimensionless. The X-axis is the waist size in cm. The relationship illustrates that small waists generally are more ellipsoidal than larger waists, which are more circular. E.g. for a waist size of approx 145 cm, a circular waist profile is assumed.

Figure 13 illustrates the measured elastic properties of the Corsinel fabric, see example 2 for materials characteristics. The figure shows the stress in N/mm as a function of the strain. The uploading (the stretch) as well as the unloading (the relaxation) is illustrated by the arrows in the figure. The uppermost, dotted curves shows the courses for the force-application in the width direction and the full curves shows the corresponding courses for the length direction. The relaxation phenomenon appears from the figure as a larger force is measured when the strain increases than when it decreases. The figure also shows that the elastic properties depend on the direction of stretch. The force at a given strain is larger when the fabric is stretched width-wise (weft direction) than length-wise (warp direction).

Figure 14 illustrates the calculated stress-strain curves of the Corsinel fabric stretched in the width (weft) or length (warp) directions when force is applied. Also shown is the average curve used for further calculations, see examples. The Y-axis is the stress in N/mm and the X-axis is the strain. The dots on the curves are the measured point while the lines are linear regressions for strain above 0.15. The uppermost, half-dotted curve represents the width direction while the lowermost, dotted curve represents the length direction. The middle full curve shows the average between the two, which is used for further calculations.

Figure 15 illustrates the calculated difference between 3 different supportive sections and the elastic section when stress-strain relationship is considered. The lowermost full curve illustrates the stress-strain relationship of the elastic section. The 3 other curves illustrate 3 different possibilities of stress-strain relationship for the supportive section. As appears from the figure the supportive section may have the same linear elastic properties as the elastic section until a lower strain limit of 0.2. Curve no. 1 illustrates one extreme that is the material becomes non-stretchable above the lower strain limit until it reaches a brittle rupture indicated at the cross. Curve no. 2 illustrates another extreme that is the material has a higher MoE than the MoE of the elastic section, which is shown by the curve being much steeper than the curve for the elastic section. Curve no. 3 illustrates an increasing

MoE, which is followed by non-stretchability. The force in the garment will be the same independent of whether the elastic section of the supportive section is considered. However, as the figure illustrates the strain will differ significantly. For example, for stress equal to 0.2 N/mm the strain of the supportive section is approx. 20-30% depending on the material while the strain in the elastic section is approx. 60 %.

Figure 16 shows the elastic properties measured on the layered material of Corsinel fabric and non-elastic mesh (material B) used as front piece. As with figures 13 and 14 the stress in N/mm as a function of the strain is shown. The uppermost, full curves shows the uploading and unloading respectively for the length direction while the lowermost, dotted curves shows the corresponding courses for the width direction. The mesh fabric is slightly oversized and hence the layered material is able to elongate for small strains. At larger strains (above 0.15-0.25) elongation is only possible by deformation of the mesh hole structure causing a steep increase in the stress-strain curve. Eventually further elongation of the layered structure becomes impossible without breaking the material.

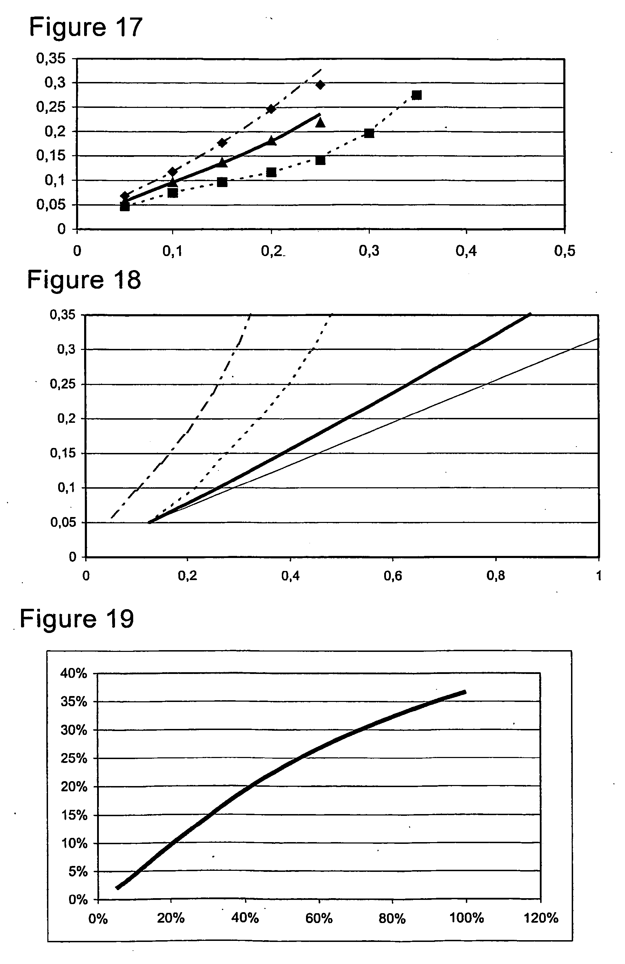

Figure 17 illustrates the stress-strain curves of the layered material stretched in the width (weft) or length (warp) directions when force is applied. Also shown is the average curve used for calculations. The Y-axis is the stress in N/mm and the X-axis is the strain. The dots on the curves are the measured point while the lines are the polynomial fits for strain above 0.07. The uppermost, half-dotted curve represents the length direction while the lowermost, dotted curve represents the width direction. The middle full curve shows the average between the two, which is used for further calculations.

Figure 18 shows the stress-strain relationship of the layered material (the uppermost, dotted curve), the elastic Corsinel material (the lowermost, thin curve) and the combination of the two for a tube (the middle full curve). The Y-axis is the calculated stress in N/mm, while the X-axis is the calculated strain. The tube used for the calculations has a total circumference of 71 cm while the width of the layered supportive section is 27.6 cm. The calculations are done as shown in example 6.

Figure 19 shows the calculated strain in the layered front piece as a function of overall strain, the strains represented in %. The calculations are done as shown in example 6. The figure demonstrates that the layered front structure stretches less in use than the elastic Corsinel fabric. It also demonstrates that the layered structure only extends to a certain elongation, any further overall elongation is then elongation of the Corsinel fabric alone.

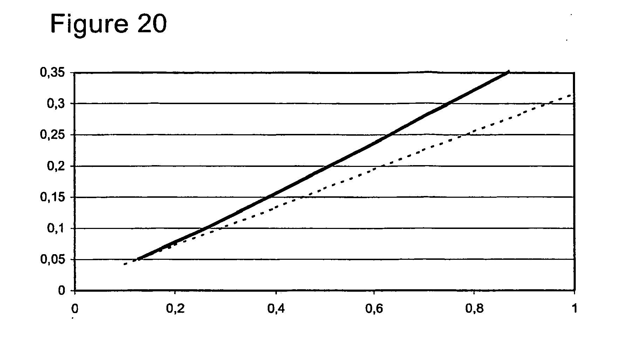

Figure 20 compares the stress in N/mm obtained at a given strain in % for a tube with the layered supportive section as shown in figure 17 (the uppermost, full curve), and a tube made entirely of the elastic Corsinel fabric (the lowermost, dotted curve). The figure demonstrates that a given stress (and hence compression) can be obtained at a lower total elongation (strain) if the tube has the less elastic front piece. A similar calculation can be made for the binder, one has to take the in-elastic hook and loop closure into account but the overall result is the same. When the binder is applied and the fabric stretched, the supportive section stretches less than the Corsinel fabric and only to a certain elongation. The supportive section allows a given stress (compression) to be obtained at a lower total elongation than needed with a binder without the less elastic front.

**Examples**

***Example 1: Tensile testing and calculation of compressing.***

It is essential that the garment provide suitable compression. However, no consensus exists on how e.g. abdominal compression is defined and how it should be measured. The torso of most humans have a somewhat ellipsoidal shape which implies that the pressure provided by e.g. an abdominal binder varies around the torso according to the LaPlace equation. Hence, a distinction between local measurements made by, e.g., a probe, and overall materials characteristics is needed. In the following, guidance is provided as to how the elastic stress is measured and related to compression as defined here.

When an elastic material is stretched or elongated the material experiences an elastic stress. This elastic stress depends on the elongation and the fundamental properties of the elastic materials. It can be measured by standard mechanical tensile testing as is well known in the art.

Tensile testing is performed by applying a given force (F) to the material and measuring the elongation ($\Delta$x) of the material as illustrated in figure 11.

The force and elongation measured depends on the size of the sample used for testing. For elastic fabrics (having a negligible thickness) one may define:

Stress, σ as the force per width of sample = $\dfrac{F}{w}$

Strain, ε as the elongation per un-stretched length of sample = $\dfrac{\Delta x}{x}$ x

The elasticity of the fabrics may then be represented by either force-elongation curves or stress-strain curves. The systematic unit of force is Newton (N) and stress is in this case stated in N/m, N/cm or N/mm. Other units of force are also used as indicated in Table 1. Strain is usually given as either a percentage (%) or in pure numbers.

**Table 1:** Conversion factors between different units of force. As an example 1 kgf is equal to 9.807 N or 2.205 lbf.

| Force | N<br>(Newton) | kgf<br>(kilo gram force) | lbf<br>(pound-force) |
|---|---|---|---|
| **1N** | 1 | 0.102 | 0.225 |
| **1 kgf** | 9.807 | 1 | 2.205 |
| **1 lbf** | 4.448 | 0.454 | 1 |

When stress is defined as force per width of sample, one may use the stress-strain relationship for linear-elastic materials for elastic fabrics as well

$$\sigma = E \cdot \varepsilon$$

where σ is the stress and ε is strain. Hence E, which usually denotes the modulus of elasticity (MoE), may represent an expression for the elasticity of the fabric, even though it is not a MoE in the normal sense. Accordingly, the MoE of elastic fabrics is in this case defined as the slope of the stress-strain curves, provided that the stress is defined as force per width of sample.

When an elastic material, such as a compression textile, is stretched across a curved surface a resulting inwardly directed force is obtained i.e. an overpressure or compression is generated. The compression ($\Delta p$) depends

on the local radius (*r*) of curvature according to $\Delta p = \dfrac{\sigma}{r}$

where σ is the stress (N/mm) in the plane of the textile at a given elongation.

The fact that the compression generated by a compression textile depends on the local radius of curvature has important implications for the design of such textiles. Most people and hence patients have an abdominal, i.e. waist circumference, which is more ellipsoidal than circular in shape. The ellipsoidal character of the waist is more pronounced in people with a small waist, whereas people with a large waist tend to be more circular in profile. This implies that people wearing a compression textile covering the waist/abdomen will experience a higher compression on or above the hips, where the radius of curvature is small, compared to on the centre of the abdomen or on the back, where the radius of curvature is larger.

More specifically, if the waist circumference is described by an ellipsoid of semi-major axis a and semi-minor axis $b$, as common in the art, then the radius of curvature varies between $r_{min}$ and $r_{max}$ given by

$$r_{\min} = \frac{b^2}{a}; r_{\max} = \frac{a^2}{b}$$

The corresponding maximum compression (at the hips) is then

$$\Delta p_{\max} = \frac{\sigma \cdot a}{b^2}$$

The minimum compression (around the midline of the abdomen or back) is

$$\Delta p_{\min} = \frac{\sigma \cdot b}{a^2}$$

Finally, the average compression can be expressed approximately by

$$\Delta p_{average} = \frac{\sigma}{\sqrt{\frac{1}{2}(a^2 + b^2)}}$$

This corresponds to the compression of a circular profile having the same perimeter as the ellipsoid defined by semi axes a and b.

Thus, a compression garment is characterized by the elastic forces provided by the fabric used to manufacture the garment. The compression obtained when using various sizes of the garment on humans is illustrated in the following sections.

The systematic unit for pressure, and hence compression, is the Pascal (Pa = $N/m^2$). However, traditionally other units such as mmHg are used.

Table 2. Conversion factors between different units of compression. As an example 1000 Pa is equal to 7.5 mmHg or 0.15 psi.

| Compression | Pa (Pascal) | mmHg (mm Mercury) | psi (pound-force pr. square inch) |
|---|---|---|---|
| 1 Pa | 1 | 0.0075 | 0.00015 |
| 1 mmHg | 133.33 | 1 | 0.0193 |
| 1 psi | 6894.76 | 51.715 | 1 |

As shown above estimation of the compression of a given abdominal compression textile depends on the size and waist profile of the user

For the further calculations a simple relationship between waist size and ellipsoidal a and b axes is assumed, which is illustrated in figure 12.

An approximate expression for the relationship is the following, where w is the waist size in cm.

$$\frac{a}{b} = 2.7972 \times 10^{-6} \times w^3 - 10.849 \times 10^{-4} \times w^2 + 0.11955 \times w - 2.051$$

*Example 2. Materials.*

**Material A: Spacer fabric**

Warp knitted spacer fabric
Construction: Double needle bar raschel fabric with a plain back side and a hole pattern on the front side (see-through).
Yarn: 100% Polyester, filament yarns and monofil pile yarn
Fabric characteristics:

Thickness: 3.0 mm (DIN EN ISO 5084)

Square meter weight 320 g/m$^2$ (ISO 3801:1997).

Compression stress, cross direction: 12.0 kPa at 40 % stretch (DIN 53 577)

This fabric is an example of a virtually non-stretchable cushioning fabric.

**Material B: Tricot fabric, mesh fabric**

Warp knitted fabric
Construction: Single needle bar warp knitted fabric with hole structure.
Yarn: 100% Polyamide, filament yarn
Fabric characteristics:

Thickness: 0,33 mm (internal test method).
2.2 holes per cm (width), 3.5 holes per cm (length).
Square meter weight 65.0 g/m$^2$ (ISO 3801:1997).

This fabric is an example of a virtually non-stretchable thin fabric.

**Material C: Santoni compression fabric**

Bodysize Santoni SM 8-8 circular weft knitted fabric
Composition: Polyamide covered Elastane yarn, 50% Polyamide, 50% Elastane.
Knitting structure: Single pique 1-1.

Pattern (X:knit, =:tuck, 0:miss):
X=X=
=X=X

*Fabric variant C1, Corsinel:*

Fabric characteristics:

Thickness: 1,09 mm (internal test method).
Stitch density: 1080 (cm$^{-2}$), 60 courses per cm and 18 wales per cm.
Square meter weight 402 g/m$^2$. (ISO 3801:1997).
Bulk density: 0.37 g/cm$^3$ (calculated).

*Fabric variant C2:*

Fabric characteristics:

Thickness: 1,24 mm (internal test method).
Stitch density: 1320 (cm$^{-2}$), 82 courses per cm and 16 wales per cm.
Square meter weight 493 g/m$^2$. (ISO 3801:1997).
Bulk density: 0.40 g/cm$^3$ (calculated).
C1 and C2 are examples of compression fabrics with high recovery "power".

**Material D: Santoni comfort fabric**

Santoni circular weft knitted fabric, washed.
Composition: 97% Polyamide, 3% Elastane (3 yarns knitted together)
Knitting structure: Single mini jacquard
Pattern (X:knit, =:tuck, 0:miss):

XX0
XX=
X0X
X=X
0XX
=XX

Fabric characteristics:

Thickness: 1.34 mm (internal test method).
Stitch density: 576 (cm$^{-2}$), 36 courses per cm and 16 wales per cm.
Square meter weight 327 g/m$^2$. (ISO 3801:1997).
Bulk density: 0.24 g/cm$^3$ (calculated).

This material is an example of an easily stretchable fabric.
Elastic properties of the materials were measured using British Standard BS 4952; 1992 using 50 mm wide samples. The results are provided in Table 3 below:

**Table 3.** Force and elongations of different materials.

| Material | Direction | Force at 40 % elongation (kgf) | Elongation at 2 kgf (%) |
|---|---|---|---|
| A | width/weft | - | 11 |
| | height/warp | - | 9 |
| B | width/weft | 1.2 | 44 |
| | height/warp | - | 20 |
| C1 | width/weft | 0.69 | 103 |
| | height/warp | 0.50 | 138 |
| | width/weft | 0.07 | 154 |
| D | height/warp | 0.11 | 130 |

Non-stretchable fabric (A or B) shows an elongation at 2 kgf below 60 % in both weft and warp directions.
A compression fabric (C1) is stretchable with an elongation at 2 kgf above 60 % in both weft and warp directions but has a high recovery power defined as a force at 40 % elongation above 0.2 kgf in both weft and warp directions.
Stretchable cushioning fabrics (D) shows an elongation at 2 kgf above 60 % in both weft and warp directions and a force at 40 % elongation below 0.2 kgf in both weft and warp directions.
Table 4 shows the composition of the compression garments, that is the pants, binder and tube.

|  | Pants | Binder | Tube |
|---|---|---|---|
| Body, Corsinel fabric | Polyamide (50 %) | Polyamide (50 %) | Polyamide (50 %) |
|  | Elastane (50 %) | Elastane (50 %) | Elastane (50 %) |
| Waist elastic | Polyamide (70-91 %) Elastane (8-9 %) w silicone coating | Polyester (31 %) Elastane (13 %) Polyamide (9 %) Cotton (24 %) Silicone (23 %) | Polyester (31 %) Elastane (13 %) Polyamide (9 %) Cotton (24 %) Silicone (23 %) |
| Strap elastic | Polyamide (83 %) Elastane (13 %) | | |
| Lace | Polyamide (79 %) Elastane (21 %) | | |
| Leg elastic | Polyamide (63 %) Polyester (19 %) Elastane (18 %) | | |
| Inner crotch | Cotton (91 %) Elastane (9 %) | | |
| Outer Crotch | Polyamide (85 %) Elastane (15 %) | | |
| Mesh | | Polyamide (100 %) | Polyamide (100 %) |
| Elastic Hook | Polyamide (100 %) | Nylon (70 %) Polyurethane (30 %) | |
| Loop fabric | Polyamide (100 %) | 100 % Nylon | |
| Thread | Polyester (100 %) | Polyester (100 %) | Polyester (100 %) |
| Label | Polyester (100 %) | Polyester (100 %) | Polyester (100 %) |
| Hole trim (optional hole) | Polyamide (50 %) Elastane (50 %) | | |
| Ribbon (optional hole) | Polyamide (100 %) | | |
| Stopper (optional hole) | Polyamide w. metal spring (100 % | | |
| Hook fabrics for prolabs | Polyamide (100 %) | | |
| Loop fabrics for prolabs | Polyamide (100 %) | | |

**Table 4:** Composition of Corsinel Pants, binder and tube.

*Example 3: Elastic Properties of the Garments I.*

In this example the supportive section is considered to be entirely non-stretchable in the calculations of the compression obtained.

The abdominal binder as shown in figure 3a was used for the tensile testing.

Test samples 1 - 6, each having a size of about 75 mm x 25 mm, were cut from an abdominal binder as indicated in Figure 3A. Every 75 mm x 25 mm sample, as representatively shown in Figure 3B, was then cut into three 25 mm x 25 mm pieces and subjected to tensile testing as shown in example 1.

Figure 4 shows the average stress-strain curves from tensile testing of the test samples marked 1 and 6 in Figure 3A, corresponding to widthwise (weft) and lengthwise (warp) elongation, respectively, of fabric variant C1 textile. The stress is defined as shown in example 1 as force per width of the sample. Figure 4 also shows that the average (width and length) stress-strain curve of fabric variant C1, a linear fit to the data from 20% ($\varepsilon$=0,2) to 200% elongation ($\varepsilon$=2,0), results in the following relation:

$$\sigma = 0.1972 \cdot \varepsilon + 0.0756$$

where $\sigma$ is the stress in N/mm and $\varepsilon$ is the elongation in absolute value. These measurements were made on small pieces of fabric cut from the actual binder and hence cannot be directly compared to the measurements of example 2 which were measured on large pieces of fabric made specifically for the test.

Figure 5 shows the average stress-strain curves of the samples marked 1 & 6, 2 & 5, 3 & 4 in Figure 3A. Samples marked 1 and 6 correspond to fabric variant C1, and samples marked 3 and 4 correspond to fabric variant C2. As expected, Figure 5 demonstrates that fabric C1 (samples 1 & 6) provides the smallest stress at a given strain and fabric C2 (samples 3 & 4) provides the highest stress at a given strain. As illustrated in example 1 the relationship between stress and compression is linear, hence fabric C1 provides the smaller compression at a given strain compared to C2.

**Compression in Abdominal Tube I**

Consider an abdominal tube made wholly of compression fabric C1, hence

$$\sigma = 0.1972 \cdot \varepsilon + 0.0756$$

It will be assumed that the user of the tube has an ellipsoidal waist profile with a=206.6 mm and b=111.7 mm. This corresponds to a waist perimeter of close to 1000 mm. If it is further assumed that the un-stretched tube has a waist perimeter of 625 mm, then the elongation is 60% when the tube is worn, and the elastic stress is 0.1939 N/mm according to the above expression. Using the assumptions and calculations shown in example 1, the following compression estimates may then be calculated:

Table 5. Compression estimates for the abdominal tube of fabric C1.

| | | |
|---|---|---|
| $\Delta p_{min}$ | 507 Pa | 3.8 mmHg |
| $\Delta p_{average}$ | 1168 Pa | 8.8 mmHg |
| $\Delta p_{max}$ | 3210 Pa | 24.1 mmHg |

**Compression in Abdominal Tube II**

Next considered is an abdominal tube similar to that of the previous section but provided with a 170 mm wide, non-stretchable supportive section. In this case the elastic part of the un-stretched textile has a width of 625 mm -170 mm = 455 mm. When stretched, the elastic part has a width of 1000 mm -170 mm = 830 mm; hence the elongation of the elastic textile is 82%. The stress in the fabric is then 0.2354 N/mm using the stress-strain expression. Compression estimates are:

Table 6. Compression estimates for the abdominal tube made of fabric C1 and including a supportive section.

| | | |
|---|---|---|
| $\Delta p_{min}$ | 613 Pa | 4.6 mmHg |
| $\Delta p_{average}$ | 1413 Pa | 10.6 mmHg |
| $\Delta p_{max}$ | 3884 Pa | 29.2 mmHg |

It is clear that the tube with supportive section provides a higher compression than a similar tube made from compression fabric only. Hence, if a given compression is needed, the use of a supportive section allows the use of a more elastic textile material and/or a smaller elongation than that needed with a wholly elastic tube.

**Compression in Abdominal Tube III**

A calculation of the compression provided by the abdominal binder of figure 3a follows along the lines indicated above. One needs to take into account that three different elastic regions (1&6, 2&5 and 3&4) are involved, as is a 170 mm wide, substantially non-stretchable, front piece. One also needs to assume an ellipsoidal waist profile of the user and estimate the a and *b* axes corresponding to a given waist perimeter (size). For the sake of simplicity, the relationship shown in Figure 12 and illustrated by the equation in example 1 will be assumed.

The table below provides calculated compression estimates for the abdominal binder of figure 3A.

**Table 7**. Compression estimates for the abdominal binder of figure 3A.

| Size | Waist (cm) | Elongation (%) | $\Delta p_{min}$ (mmHg) | $\Delta p_{average}$ (mmHg) | $\Delta p_{max}$ (mmHg) |
|------|------------|----------------|-------------------------|------------------------------|--------------------------|
| XS | 85 - 95 | 58 - 82 | 4.5-5.4 | 12.0-13.5 | 36.0-37.1 |
| S | 94 - 108 | 54 - 82 | 4.2-5.3 | 10.5-12.0 | 31.0-31.4 |
| M | 105 - 120 | 54 - 81 | 4.0-5.9 | 9,3-10.6 | 19.2-23.5 |
| L | 115 - 132 | 53 - 80 | 4.3-6.6 | 8.5-9.6 | 14.6-17.8 |
| XL | 126 - 145 | 54 - 80 | 4.8-8.7 | 7.8-8.7 | 8.7 |

**Compression in Undershirt**

The results of similar calculations relating to the undershirt of figures 6A and 6B are presented in the table below:

**Table 8**. Compression estimates for the undershirt of figures 6A and 6B.

| Size | Waist (cm) | Elongation (%) | $\Delta p_{min}$ (mmHg) | $\Delta p_{average}$ (mmHg) | $\Delta p_{max}$ (mmHg) |
|------|------------|----------------|-------------------------|------------------------------|--------------------------|
| XS | 85 - 92 | 45 - 60 | 3.8-4.4 | 10.2-11.2 | 30.7-32.9 |
| S | 90 - 105 | 35 - 63 | 3.3-4-4 | 8.5-10.2 | 24.5-25.7 |
| M | 100 - 115 | 36 - 60 | 3.2-4.6 | 7.8-9.0 | 18.7-20.5 |
| L | 110 - 125 | 37 - 60 | 3.5-5.1 | 7.2-8.3 | 14.0-16.5 |
| XL | 120 - 145 | 37 - 71 | 3.7-8.0 | 6.6-8.0 | 8.0 |

***Example 4: Test of calculations***

An abdominal tube was made of compression fabric C with a stress-strain relationship of

$$\sigma = 0.2156 \cdot \varepsilon + 0.0869$$

as measured by tensile testing. The tube included a 170 mm wide, substantially non-stretchable, front piece made of material A. The tube had a total un-stretched waist size of 860 mm (elastic width 690 mm) and was mounted on a cylindrical plastic tube of 400 mm in diameter. The perimeter of the tube was 1256.6 mm, corresponding to an elongation of 57% of the elastic material. The calculated stress was 0.210 N/mm and the calculated compression was 7.8 mmHg everywhere (circular profile).

To test the calculation, a pressure sensor was inserted below the front piece of the abdominal tube between the abdominal binder and the plastic tube and the compression was measured directly. The equipment used was the I-SCAN® Lite Pressure Measurement System from Tekscan, Inc., 307 West First Street, South Boston, USA, comprising a type 5101 sensor in the form of a rectangular shaped matrix (112 mm x 112 mm) forming squares and resulting in a 44 x 44 grid (1936 active measuring cells). Frames were captured, exported to a spreadsheet and averaged.

The measured compression in this case was approximately 7 mmHg, which is considered in good agreement with the calculated value.

***Example 5: Supportive and elastic sections.***

The garments of the invention all comprise a knitted tubular compression section, which comprises an elastic section and a supportive section. In this example measurements on the linear elastic section (Corsinel or material C1, see example 2) as well as measurements of the supportive or progressive elastic section (layered material consisting of one layer of Corsinel, material C1 and one layer of mesh fabric, tricot fabric, material B, see example 2) were used as basis for the calculations. During the measurements the layered materials had the same size.

**Test setup**

Force-elongation curves were obtained by tensile testing of test samples following BS 4952 1996, section 2.1.

**Table 9**: Test conditions for tensile testing

| Sample width | 5 cm |
|---|---|
| Point of load application cycle | 3 N/cm |
| Data collected on | 2nd cycle |
| Temperature | Room temperature (22-23 °C) |
| Acclimatised room | No |
| Equilibration of samples | No |

**Elastic properties, elastic section**

Figure 13 shows the elastic properties of the Corsinel fabric, depending on the direction of stretch and whether it is during uploading or unloading. The lowermost full curves show the data of the length direction while the uppermost dotted curves shows the data for the width direction. The measured elastic properties during uploading are used for the linear regression curves shown in figure 14. The figure also shows the actual measure points used for the linear regression. Again the lowermost curve represent the length direction and the uppermost the width direction. The middle full curve represents the average between the two. The linear regression is done using the linear regression function in the Excel ® spreadsheet.

The linear functions appear from Table 10.

**Table 10**. Fitted stress-strain relationships for Corsinel fabric.

| Direction | Fit |
|---|---|
| Width | Stress, $\sigma$ (N/mm) = 0.355 * Strain, $\varepsilon$ + 0.0100 |
| Length | Stress, $\sigma$ (N/mm) = 0.261 * Strain, E + 0.0105 |
| Average | Stress, $\sigma$ (N/mm) = 0.305 * Strain, $\varepsilon$ + 0.0117 |

The Corsinel fabric is equal to the fabric C1, which was used in relation with example 3. The difference in the equations may be due to the difference in test set-up, e.g. sample size, measuring on second cycle as opposed to the first cycle and so forth.
The elastic section has linear-elastic material properties as defined in example 1.

**Elastic properties, supportive section.**

The supportive section has progressive elastic material properties, which, as explained earlier, may have similar strain as the elastic section until a lower strain limit of about 20%. In one extreme situation the supportive section is entirely non-stretchable above the lower strain limit. In another extreme situation the supportive section has a higher elasticity above the lower strain limit as expressed by the definition of the MoE as illustrated in example 1. In the preferred embodiment the supportive section has an increasing elasticity as expressed by the definition of the MoE between the lower strain limit and an upper strain limit of about 40%, following which, the fabric becomes entirely non-stretchable.
Figure 15 illustrates different embodiments of the supportive section. The figures shows an example of the stress-strain curve for the elastic material and three different supportive sections, one being non-stretchable above lower strain limit (curve no. 1), one having a higher MoE than the elastic section (curve no. 2) and one having an increasing MoE (curve no. 3). The cross indicates brittle rupture at a certain stress occurring for the non-stretchable material (no. 1) and the material having an increasing MoE (no. 3).

One embodiment of making a material with progressive elastic material properties is to make a layered material with a first fabric layer of an elastic material and a second oversized layer of another material. The second oversized layer may be made of materials having an MoE which is much higher than the MoE of the first fabric, this would correspond to curve no. 2 in figure 15. In another embodiment the second oversized layer may be made of a completely non-stretchable fabric (corresponding to curve no. 1) and in yet another embodiment the second oversized layer may be made of a fabric having an increasing MoE until it reaches a point where it is completely un-stretchable - corresponding to curve no. 3. Accurately adjustment of the over-sizing or slack in the material makes it possible to control the lower strain limit from which the material changes properties.

Another embodiment of a material with changing properties is a material made of a fabric, which is completely un-stretchable but where the material is provided with a certain amount and size of holes. The holes will have the effect of making the fabric able to stretch relatively freely up to a limit where the holes are beginning to deform following which the MoE will increase as the holes are deforming. Finally the holes are completely deformed and the material will not be able to stretch further.

This kind of material may also be used as the second oversized layer in the layered material. In this case it will make a smooth transition over the lower strain limit while still provide the complete control at the upper strain limit due to the non-stretchability of the material occurring when the holes are completely deformed.

Figures 16 and 17 show the elastic properties of the layered material, which in the construction of the garments is used as the supportive section. The layered material used in the test has the same dimensions in the layers meaning that the delaying effect of the oversizing of one of the layers is not illustrated. Corresponding to figures 13 and 14, figure 16 shows the results of the actual measurements and figure 17 shows the result as fitted by polynomial fit. In figure 16 the lowermost dotted curves show the measurements in the width direction, while the uppermost full curves show the measurements in the length direction. The arrows indicate the uploading (the stretch) and the unloading (the relaxation). In figure 17 the lowermost dotted curve represent the polynomial fit of the results in the width direction, the uppermost half-dotted curve represent the fit of the results in the length direction and the middle full curve represents the fit of the results as an average. The points are the actual measurements.

The polynomials appear from Table 11.

Table 11. Fitted stress-strain relationships for supportive section (force application). Stress (N/mm): σ, strain (absolute value): ε

| Direction | Fit |
| --- | --- |
| Width | $\sigma = 5.0 \times \varepsilon^4 + 10.0 \times \varepsilon^3 - 5.2 \times \varepsilon^2 + 1.19 \times \varepsilon - 0.00114$ |
| Length | $\sigma = 2.1 \times \varepsilon^2 + 0.661 \times \varepsilon + 0.0308$ |
| Average | $\sigma = 3.0 \times \varepsilon^4 + 5.0 \times \varepsilon^3 - 1.6 \times \varepsilon^2 + 0.926 \times \varepsilon + 0.015$ |

***Example 6: Elastic Properties of Garments II.***

**Elastic properties of the tube and binder**

The stress-strain relationship of the tube can be calculated combining the corresponding relationships for the elastic section and the supportive section. The force in the compression section must be same independent of whether the elastic section or the supportive section is considered. In the following we will disregard any effect of the waist elastic and simply assume that it follows the elastic or supportive section respectively, when they stretch. We will use the average (width/length) stress-strain curves (force application) to calculate the stresses in the Corsinel fabric, the elastic section and the layered structure, the supportive section, when the binder or tube is stretched.

The difference in strain in the supportive section and the elastic section is illustrated in figure 15. For example, for stress equal to 0.2 N/mm the strain of the supportive section is approx. 20-30% depending on the material while the strain in the elastic section is 60 %.

The total elongation may be found as the sum of the elongations of the separate sections, where each elongation is the strain times the length of the section. So the following conditions apply:

- The force in the elastic section is equal to the force in the supportive section.

- The total elongation is equal to the sum of the elongations of the separate sections.

- The elongation of a section is equal to the strain times the length of the section.

$$F_{\text{elastic}} = F_{\text{supportive}}$$

$$\Delta x_{total} = \Delta x_{\text{elastic}} + \Delta x_{\text{supportive}}$$

$$\Delta x_{\text{section}} = \varepsilon_{\text{section}} \cdot l_{\text{section}}$$

Using these conditions, it is possible to calculate numerically how a total elongation (strain) is distributed between the elastic section and the supportive section. The tube is assumed to have a total circumference of 71 cm (=710mm) and a layered supportive section of a width of 27.6 cm (=276mm) leaving an elastic section of 43.4 cm (=434mm). One has to take into consideration that the supportive section may have linear elastic material properties until strain of 20% (0.2). Provided that the material within this limit (below strain of 20%) has properties like the elastic section corresponding to the Corsinel fabric, the stress at 20% is equal to 0,07 N/mm, see figure 14. That means that only stress values above 0,07 N/mm causes a strain in the supportive section following the polynomial fit of Table 11.

Table 12 shows calculations of strain and elongations in the elastic section and the supportive section. Calculations of the total elongation in the tube are also illustrated.

**Table 12.** Calculation of strain and total elongation in the tube.

| | σ = 0.05 N/mm | σ=0.10 N/mm | σ=0.15 N/mm | σ = 0.20 N/mm | σ = 0.25 N/mm | σ = 0.30 N/mm |
|---|---|---|---|---|---|---|
| $\varepsilon_{\text{elastic}}$ $\Delta x_{\text{elastic}}$ (mm) | 0,1255 54,5 | 0,2896 125,7 | 0,4536 196,9 | 0,6176 268,1 | 0,7817 339,2 | 0,9457 410,4 |
| $\varepsilon_{\text{supportive}}$ $\Delta x_{\text{supportive}}$ (mm) | 0,1255 34,7 | 0,2138 59,0 | 0,2748 75,8 | 0,3392 93,6 | 0,3964 109,4 | 0,4428 122,2 |
| $\Delta x_{\text{total}}$ (mm) | 89,1 | 184,7 | 272,7 | 361,7 | 448,6 | 532,6 |

By dividing the total elongation by the initial length of the binder (710mm) it is possible to calculate a value for the total strain in the tube. The result of the calculations is shown in figure 18, where the thin curve illustrates the strain in the elastic section, the dotted curve illustrates the strain in the supportive section and the full curve illustrates the strain in the tube.

Using the fitted polynomial curve of Table 11 and the linear regression of Table 10 it is possible to calculate the strain in the layered supportive section as a function of the overall strain. Figure 19 shows the calculated strain in the supportive section as a function of overall strain. The figure demonstrates that the supportive section stretches less in use than the elastic section. It also demonstrates that the supportive section only stretches to a certain elongation, any further overall elongation in the tube is then elongation of elastic section alone. Figure 20 compares the stress obtained at a given strain for a tube with the front piece as described above, and a tube made entirely of elastic material, e.g. Corsinel, is compared compared, the full curve illustrating the tube with the supportive section and the dotted curve illustrating the elastic section. For instance a strain value of 50% results in a stress value of 0.20 N/mm when the tube according to the invention is considered and only 0.16 N/mm when the all-elastic tube is considered. The figure demonstrates that a given stress (and hence compression) can be obtained at a lower total elongation (strain) if the tube has the less elastic front piece. In both cases the circumference of the tube is 71 cm, and for the combination material the supportive section has a width of 27.6 cm.

**Force-compression relationship**

We will consider a simple one-dimensional model calculating the compression around the abdomen disregarding the effects of different heights of the garments. Assuming a given total circumference of the binder or tube, a given waist size of the user and using the waist profile relationship from figure 12 compression estimates are calculated as indicated in example 1.

In case of the binder, one needs to take the hook and loop fabric into account; which account for between 3 and 10 cm of the circumference depending on how tight the binder is adjusted. The hook and loops fabrics are fully in-elastic, hence all elongation of the binder is distributed between the Corsinel fabric and the supportive front section.

**Sizes of the binder and tube.**

The binder comes in two heights (low and high) and five sizes (XS, S, M, L, XL): Parameters (after wash at 80C°) are given in Table 13.

**Table 13:** Parameters for the binder (all numbers in cm)

| Binder | XS | S | M | L | XL |
|---|---|---|---|---|---|
| Circumference tight | 57 | 63 | 69 | 75 | 80 |
| Circumference loose | 63 | 69 | 75 | 80 | 86 |
| Width of sandwich | 28.5 | 29.9 | 31.5 | 33.1 | 33.9 |
| Width of mesh fabric | 29.5 | 31.3 | 33.9 | 36.1 | 37.1 |
| Width of hook fabric | 3 | 3 | 3 | 3 | 3 |
| Width of loop fabric | 10 | 10 | 10 | 10 | 10 |
| Waist (user) | < 87 | 86-95 | 94-103 | 102-109 | >109 |
| | | | | | |
| | XS | S | M | L | XL |
| **High version** | | | | | |
| Height, front | 26.5 | 27.5 | 28.5 | 31.5 | 34.5 |
| Height, back | 20 | 21 | 22 | 23 | 24 |
| **Low version** | | | | | |
| Height, front | 19.5 | 20.5 | 21.5 | 24.5 | 27.5 |
| Height, back | 13 | 14 | 15 | 16 | 17 |

The tube is identical to the binder except that the initial knitted seamless tube has not been cut and provided with the hook and loop closure. Otherwise the tube is provided with the same sandwich front and provided with easily extendible elastic trims with a silicon pattern on the side facing the skin of the user.

The tube comes in one height and five sizes (XS, S, M, L, XL): Parameters (after wash at 80C°) are given in Table 14.

**Table 14:** Parameters for the tube (all numbers in cm)

| Tube | XS | S | M | L | XL |
|---|---|---|---|---|---|
| Circumference | 57 | 64 | 71 | 78 | |
| Height, front | 21.5 | 22.5 | 23.5 | 26.5 | |
| Height, back | 15 | 16 | 17 | 18 | |
| Width of sandwich | 24.4 | 25.8 | 27.6 | 28.8 | 30.3 |
| Width of mesh fabric | 26 | 27.6 | 29.8 | 31.6 | 33.5 |
| Waist (user) | < 87 | 86-95 | 94-103 | 102-109 | >109 |

**Compression**

Table 15 provides the calculated average compressions for the tube, while Table 16 provides the calculated maximum and minimum compressions for the tube. The unit for the compression is mmHg.

| Waist (cm) | XS | S | M | L | XL |
|---|---|---|---|---|---|
| 78 | 10,04 | 6,14 | 3,17 | | |
| 86,5 | 12,61 | 8,48 | 5,46 | 3,05 | 1,05 |
| 94,5 | 14,75 | 10,40 | 7,26 | 4,80 | 2,82 |
| 102,5 | 16,64 | 12,11 | 8,83 | 6,28 | 4,29 |
| 109 | 18,02 | 13,36 | 9,99 | 7,36 | 5,32 |
| 119 | 19,90 | 15,08 | 11,59 | 8,84 | 6,71 |
| 135 | 22,40 | 17,39 | 13,76 | 10,86 | 8,62 |

Table 15. Calculated average compressions for the tube ($\Delta p_{average}$) in mmHg.

| Waist (cm) | XS | | S | | M | | L | | XL | |
|---|---|---|---|---|---|---|---|---|---|---|
| | min | max | min | max | min | max | min | max | min | max |
| 78 | 3,97 | 31,77 | 2,43 | 19,43 | 1,25 | 10,03 | | | | |
| 86,5 | 5,04 | 39,27 | 3,39 | 26,42 | 2,18 | 17,01 | 1,22 | 9,49 | 0,42 | 3,27 |
| 94,5 | 6,13 | 43,30 | 4,32 | 30,52 | 3,02 | 21,31 | 1,99 | 14,09 | 1,17 | 8,27 |
| 102,5 | 7,39 | 44,35 | 5,38 | 32,26 | 3,92 | 23,53 | 2,79 | 16,75 | 1,90 | 11,42 |
| 109 | 8,61 | 43,28 | 6,39 | 32,09 | 4,78 | 23,99 | 3,52 | 17,67 | 2,54 | 12,77 |
| 119 | 11,02 | 39,17 | 8,36 | 29,69 | 6,42 | 22,82 | 4,90 | 17,40 | 3,72 | 13,21 |
| 135 | 17,22 | 29,61 | 13,37 | 23,00 | 10,58 | 18,20 | 8,35 | 14,36 | 6,62 | 11,39 |

Table 16: Calculated minimum and maximum compressions for the tube ($\Delta p_{min}$ and $\Delta p_{max}$) in mmHg

The average compression varies between 0 and 22 mmHg, but if the sizing recommendations are followed, average compressions between 5 and 13 mmHg are obtained.

| Waist (cm) | XS | | S | | M | | L | | XL | |
|---|---|---|---|---|---|---|---|---|---|---|
| | loose | tight | loose | tight | loose | tight | loose | tight | loose | tight |
| 78 | 7,41 | 9,87 | 4,32 | 6,55 | 1,83 | 3,91 | | | | |
| 86,5 | 10,12 | 12,35 | 6,91 | 8,88 | 4,39 | 6,19 | 2,62 | 4,00 | 0,80 | 2,42 |
| 94,5 | 12,36 | 14,37 | 8,96 | 10,77 | 6,35 | 7,99 | 4,58 | 5,76 | 2,75 | 4,17 |
| 102,5 | 14,32 | 16,14 | 10,78 | 12,43 | 8,06 | 9,56 | 6,23 | 7,26 | 4,34 | 5,64 |
| 109 | 15,74 | 17,42 | 12,10 | 13,64 | 9,30 | 10,71 | 7,43 | 8,35 | 5,49 | 6,69 |
| 119 | 17,68 | 19,14 | 13,91 | 15,28 | 11,00 | 12,28 | 9,06 | 9,85 | 7,03 | 8,12 |
| 135 | 20,24 | 21,42 | 16,33 | 17,46 | 13,31 | 14,38 | 11,28 | 11,87 | 9,11 | 10,06 |

Table 17. Calculated average compressions for the binder ($\Delta p_{average}$) in mmHg.

The average compression varies between 0 and 21 mmHg, but if the sizing recommendations are followed, average compressions between 5 and 12 mmHg are obtained.

The foregoing descriptions and drawings should be considered as illustrative only of the principles of the invention. The invention may be configured in a variety of shapes and sizes and is not limited by the dimensions of the preferred embodiment. Numerous applications of the present invention will readily occur to those skilled in the art. Therefore, it is not desired to limit the invention to the specific examples disclosed or the exact construction and operation shown and described. Rather, all suitable modifications and equivalents may be resorted to, falling within the scope of the claims.

**Claims**

1.  A compression garment comprising a material having two sections, a linear elastic section being made of a material with linear elastic properties, and a progressive elastic section being made of a material with progressive elastic properties, said progressive elastic section having linear elastic material properties below 20% extension.

2.  The compression garment according to claim 1 wherein the progressive elastic section is made of a material type with non-linear elastic properties with an increasing modulus of elasticity, MoE, within an extension of 20% - 40% compared with the MoE corresponding to an extension below 20%, said material type being non-stretchable within an extension above 40% with a brittle rupture at increased force, and force required for rupture being higher than 0.5N/mm.

3.  The compression garment according to claim 1 wherein the progressive elastic section is made of a material type with different linear elastic properties depending on the extension, said material within an extension above 20% having linear elastic material properties with an MoE at least three times higher than the MoE of the material within an extension below 20%.

4.  The compression garment according to any of the previous claims, wherein the progressive elastic section is made of a layered material with layers having different material properties, a first layer having linear elastic material properties and another layer being slightly oversized compared to the first layer.

5.  The compression garment according to claim 4 where the material of the first layer corresponds to the material of the first section.

6.  The compression garment according to claims 4-5 wherein the oversized layer includes a virtually non-stretchable material.

7.  The compression garment according to claim 6, wherein the oversized layer of virtually non-stretchable material has holes.

8.  The compression garment according to claim 7, wherein the oversized layer of virtually non-stretchable material has holes corresponding to 25 % - 50 % of the area of the oversized layer.

9.  The compression garment according to claims 6-8, wherein the virtually non-stretchable material is made of polyamide filament yarn.

10. The compression garment according to claim 4, wherein the oversized layer includes a linear elastic material with a MoE of at least three times the MoE of the elastic section.

11. The compression garment according to any of the previous claims, wherein the linear elastic section comprises subsections with different linear elastic properties.

12. The compression garment according to any of the previous claims, wherein the linear elastic section is linear elastic at least up to an extension of 100%.

13. The compression garment according to any of the previous claims, wherein said garment is configured to produce a compression of between 1 mmHg and 40 mmHg. (133 Pa and 5330 Pa)

14. The compression garment according to any of the previous claims, wherein the garment is in the shape of an undershirt.

15. The compression garment according to any of the previous claims, wherein the garment is in the shape of an abdominal binder.

16. The compression garment according to any of the previous claims, wherein the garment is in the shape of underpants.

17. The compression garment according to any of the previous claims, wherein the garment is in the shape of boxer briefs.

18. The compression garment according to any of the previous claims, wherein the garment is in the shape of a tube, such as circular knit tube.

19. The compression garment according to any of the previous claims, wherein the garment is a medical bandage.

20. The compression garment according to any of the previous claims, wherein the width of the supportive section is at least 6 cm, such as at least 10 cm, at least 15 cm or at least 20 cm.

21. The compression garment according to any of the previous claims, wherein the width of the supportive section is less than 40 cm.

22. The compression garment according to any of the previous claims, further comprising a structure for measuring elongation.

23. The compression garment according to any of the previous claims, further comprising an elastic rubber trim.


**Patentansprüche**

1. Kompressionskleidungsstück, welches ein Material umfasst, welches zwei Abschnitte, einen linear elastischen Abschnitt, welcher aus einem Material mit linear elastischen Eigenschaften hergestellt ist, und einen progressiv elastischen Abschnitt aufweist, welcher aus einem Material mit progressiv elastischen Eigenschaften hergestellt ist, wobei der progressiv elastische Abschnitt linear elastische Eigenschaften unterhalb einer Extension von 20 % aufweist.

2. Kompressionskleidungsstück gemäß Anspruch 1, wobei der progressiv elastische Abschnitt aus einem Materialtyp mit nicht-linear elastischen Eigenschaften mit einem zunehmenden Elastizitätsmodul MoE innerhalb einer Extension von 20 % bis 40 % verglichen mit dem MoE entsprechend einer Extension unterhalb von 20 % hergestellt ist, wobei der Materialtyp nicht dehnbar innerhalb einer Extension oberhalb von 40 % ist, mit einem Sprödbruch bei erhöhter Kraft, und wobei die Kraft, welche für den Bruch erforderlich ist, höher als 0,5 N/mm ist.

3. Kompressionskleidungsstück gemäß Anspruch 1, wobei der progressiv elastische Abschnitt aus einem Materialtyp mit unterschiedlichen linear elastischen Eigenschaften in Abhängigkeit von der Extension hergestellt ist, wobei das Material innerhalb einer Extension oberhalb von 20 % linear elastische Eigenschaften mit einem MoE von zumindest drei Mal höher als der MoE des Materials innerhalb einer Extension unterhalb von 20 % aufweist.

4. Kompressionskleidungsstück gemäß irgendeinem der vorherigen Ansprüche, wobei der progressiv elastische Abschnitt aus einem geschichteten Material mit Schichten hergestellt ist, welche unterschiedliche Materialeigenschaften aufweisen, wobei eine erste Schicht linear elastische Materialeigenschaften aufweist, und eine weitere Schicht etwas überdimensioniert verglichen mit der ersten Schicht ist.

5. Kompressionskleidungsstück gemäß Anspruch 4, wobei das Material der ersten Schicht dem Material des ersten Abschnitts entspricht.

6. Kompressionskleidungsstück gemäß den Ansprüchen 4 bis 5, wobei die überdimensionierte Schicht ein nahezu nicht dehnbares Material enthält.

7. Kompressionskleidungsstück gemäß Anspruch 6, wobei die überdimensionierte Schicht aus nahezu nicht dehnbarem Material Löcher aufweist.

8. Kompressionskleidungsstück gemäß Anspruch 7, wobei die überdimensionierte Schicht aus nahezu nicht dehnbarem Material Löcher aufweist, welche 25 % bis 50 % des Bereichs der überdimensionierten Schicht entsprechen.

9. Kompressionskleidungsstück gemäß den Ansprüchen 6 bis 8, wobei das nahezu nicht dehnbare Material aus einem Polyamidfasergarn hergestellt ist.

10. Kompressionskleidungsstück gemäß Anspruch 4, wobei die überdimensionierte Schicht ein linear elastisches Material mit einem MoE von zumindest drei Mal dem MoE des elastischen Abschnitts enthält.

**11.** Kompressionskleidungsstück gemäß irgendeinem der vorherigen Ansprüche, wobei der linear elastische Abschnitt Unterabschnitte mit unterschiedlichen linear elastischen Eigenschaften umfasst.

**12.** Kompressionskleidungsstück gemäß irgendeinem der vorherigen Ansprüche, wobei der linear elastische Abschnitt linear elastisch zumindest bis zu einer Extension von 100 % ist.

**13.** Kompressionskleidungsstück gemäß irgendeinem der vorherigen Ansprüche, wobei das Kleidungsstück konfiguriert ist, um eine Kompression zwischen 1 mmHg und 40 mmHg (133 Pa und 5330 Pa) zu erzeugen.

**14.** Kompressionskleidungsstück gemäß irgendeinem der vorherigen Ansprüche, wobei das Kleidungsstück in der Form eines Unterhemds ist.

**15.** Kompressionskleidungsstück gemäß irgendeinem der vorherigen Ansprüche, wobei das Kleidungsstück in der Form einer Abdominalbinde ist.

**16.** Kompressionskleidungsstück gemäß irgendeinem der vorherigen Ansprüche, wobei das Kleidungsstück in der Form einer Unterhose ist.

**17.** Kompressionskleidungsstück gemäß irgendeinem der vorherigen Ansprüche, wobei das Kleidungsstück in der Form von Boxershorts ist.

**18.** Kompressionskleidungsstück gemäß irgendeinem der vorherigen Ansprüche, wobei das Kleidungsstück in der Form von einer Röhre ist, beispielsweise eine kreisförmige gewirkte Röhre.

**19.** Kompressionskleidungsstück gemäß irgendeinem der vorherigen Ansprüche, wobei das Kleidungsstück eine medizinische Bandage ist.

**20.** Kompressionskleidungsstück gemäß irgendeinem der vorherigen Ansprüche, wobei die Breite des stützenden Abschnitts zumindest 6 cm ist, beispielsweise zumindest 10 cm, zumindest 15 cm oder zumindest 20 cm.

**21.** Kompressionskleidungsstück gemäß irgendeinem der vorherigen Ansprüche, wobei die Breite des stützenden Abschnitts weniger als 40 cm ist.

**22.** Kompressionskleidungsstück gemäß irgendeinem der vorherigen Ansprüche, weiterhin eine Struktur zum Messen einer Ausdehnung umfassend.

**23.** Kompressionskleidungsstück gemäß irgendeinem der vorherigen Ansprüche, weiterhin einen elastischen Gummirand umfassend.

**Revendications**

**1.** Vêtement de compression comprenant un matériau ayant deux sections, une section élastique linéaire qui est réalisée avec un matériau ayant des propriétés élastiques linéaires, et une section élastique progressive qui est réalisée avec un matériau ayant des propriétés élastiques progressives, ladite section élastique progressive ayant des propriétés de matériau élastiques linéaires inférieures à 20 % d'extension.

**2.** Vêtement de compression selon la revendication 1, dans lequel la section élastique progressive est réalisée avec un type de matériau ayant des propriétés élastiques non linéaires avec un module d'élasticité croissant MoE, ayant une extension de l'ordre de 20 % à 40 % par rapport au MoE correspondant à une extension inférieure à 20 %, ledit type de matériau n'étant pas extensible dans une plage d'extension supérieure à 40 % avec une rupture fragile à une force accrue, et la force requise pour la rupture étant supérieure à 0,5 N/mm.

**3.** Vêtement de compression selon la revendication 1, dans lequel la section élastique progressive est réalisée avec un type de matériau ayant des propriétés élastiques linéaires différentes dépendant de l'extension, ledit matériau ayant une plage d'extension supérieure à 20 % avec des propriétés de matériau élastiques linéaires avec un MoE au moins trois fois supérieur au MoE du matériau dans une plage d'extension inférieure à 20 %.

**4.** Vêtement de compression selon l'une quelconque des revendications précédentes, dans lequel la section élastique progressive est réalisée avec un matériau en couche avec des couches ayant différentes propriétés de matériau, une première couche ayant des propriétés de matériau élastiques linéaires et une autre couche étant légèrement surdimensionnée par rapport à la première couche.

**5.** Vêtement de compression selon la revendication 4, dans lequel le matériau de la première couche correspond au matériau de la première section.

**6.** Vêtement de compression selon les revendications 4 et 5, dans lequel la couche surdimensionnée comprend un matériau pratiquement non extensible.

**7.** Vêtement de compression selon la revendication 6, dans lequel la couche surdimensionnée en matériau pratiquement non extensible a des trous.

**8.** Vêtement de compression selon la revendication 7, dans lequel la couche surdimensionnée en matériau pratiquement non extensible a des trous correspondant à 25 % - 50 % de la surface de la couche surdimensionnée.

**9.** Vêtement de compression selon les revendications 6 à 8, dans lequel le matériau pratiquement non extensible est réalisé à partir d'un fil continu en polyamide.

**10.** Vêtement de compression selon la revendication 4, dans lequel la couche surdimensionnée comprend un matériau élastique linéaire avec un MoE représentant au moins trois fois le MoE de la section élastique.

**11.** Vêtement de compression selon l'une quelconque des revendications précédentes, dans lequel la section élastique linéaire comprend des sous-sections avec différentes propriétés élastiques linéaires.

**12.** Vêtement de compression selon l'une quelconque des revendications précédentes, dans lequel la section élastique linéaire est élastique de manière linéaire au moins jusqu'à une extension de 100 %.

**13.** Vêtement de compression selon l'une quelconque des revendications précédentes, dans lequel ledit vêtement est configuré pour produire une compression comprise entre 1 mmHg et 40 mmHg (133 Pa et 5330 Pa).

**14.** Vêtement de compression selon l'une quelconque des revendications précédentes, dans lequel le vêtement se présente sous la forme d'un maillot de corps.

**15.** Vêtement de compression selon l'une quelconque des revendications précédentes, dans lequel le vêtement se présente sous la forme d'un bandage abdominal.

**16.** Vêtement de compression selon l'une quelconque des revendications précédentes, dans lequel le vêtement se présente sous la forme d'un slip.

**17.** Vêtement de compression selon l'une quelconque des revendications précédentes, dans lequel le vêtement se présente sous la forme d'un caleçon.

**18.** Vêtement de compression selon l'une quelconque des revendications précédentes, dans lequel le vêtement se présente sous la forme d'un tube, tel qu'un tube en jersey circulaire.

**19.** Vêtement de compression selon l'une quelconque des revendications précédentes, dans lequel le vêtement est un bandage médical.

**20.** Vêtement de compression selon l'une quelconque des revendications précédentes, dans lequel la largeur de la section de support est d'au moins 6 cm, telle qu'au moins 10 cm, au moins 15 cm ou au moins 20 cm.

**21.** Vêtement de compression selon l'une quelconque des revendications précédentes, dans lequel la largeur de la section de support est inférieure à 40 cm.

**22.** Vêtement de compression selon l'une quelconque des revendications précédentes, comprenant en outre une structure pour mesurer l'allongement.

**23.** Vêtement de compression selon l'une quelconque des revendications précédentes, comprenant en outre une bande de bordure en caoutchouc élastique.

# Figure 1

## Figure 2A

## Figure 2B

## Figure 3A

## Figure 3B

Figure 4

Figure 5

Figure 6B

Figure 6A

Figure 7A

Figure 7B

220

250

220

255

220

290

210

EP 1 959 880 B1

35

Figure 8A

Figure 8B

Figure 8C

EP 1 959 880 B1

Figure 9C

Figure 9A

Figure 9B

Figure 10A

Figure 10B

494

492

494

493

494

494

494

494

493

492

494

442

494

492

493

494

493

492

494

# Figure 11

# Figure 12

# Figure 13

## Figure 14

## Figure 15

## Figure 16

## Figure 17

## Figure 18

## Figure 19

Figure 20

**EP 1 959 880 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0024349 A
- FR 2039950
- US 2004127881 A